Europäisches Patentamt

European Patent Office

Office européen des brevets

(19)

(11)     EP 1 034 422 B1

(12)     FASCICULE DE BREVET EUROPEEN

(45) Date de publication et mention
de la délivrance du brevet:
**28.01.2004 Bulletin 2004/05**

(21) Numéro de dépôt: **98956961.1**

(22) Date de dépôt: **26.11.1998**

(51) Int Cl.⁷: **G01N 21/51**, G01N 21/64,
G01N 21/31, G01N 21/03,
G01N 33/483

(86) Numéro de dépôt international:
**PCT/FR1998/002542**

(87) Numéro de publication internationale:
**WO 1999/027349 (03.06.1999 Gazette 1999/22)**

(54) **APPAREIL ET PROCEDE DE MESURE DE PROPRIETES OPTIQUES PAR CONTROLE RETROACTIF**

VORRICHTUNG UND VERFAHREN FÜR DIE MESSUNG OPTISCHER EIGENSCHAFTEN MITTELS RÜCKWIRKENDER KONTROLLE

APPARATUS AND METHOD FOR MEASURING OPTICAL PROPERTIES BY FEEDBACK CONTROL

(84) Etats contractants désignés:
**AT BE CH CY DE DK ES FI FR GB GR IE IT LI LU MC NL PT SE**

(30) Priorité: **26.11.1997 FR 9714864**

(43) Date de publication de la demande:
**13.09.2000 Bulletin 2000/37**

(60) Demande divisionnaire:
**03022315.0**
**03022389.5**

(73) Titulaire: **INSTITUT PASTEUR**
**75724 Paris Cédex 15 (FR)**

(72) Inventeurs:
• **GAILLON, Laurent**
**F-75015 Paris (FR)**
• **LONGIN, Robert**
**F-92170 Vanves (FR)**
• **LUU, Thanh, Dung**
**F-78180 Montigny le Bretonneux (FR)**

(74) Mandataire: **Michelet, Alain et al**
**Cabinet Harlé et Phélip**
**7, rue de Madrid**
**75008 Paris (FR)**

(56) Documents cités:
EP-A- 0 303 524       WO-A-92/07245
WO-A-92/13482         US-A- 3 731 806
US-A- 4 058 363       US-A- 4 447 150
US-A- 4 622 468       US-A- 4 740 356

## Description

La présente invention est relative à un appareil et à un procédé de mesure de propriétés optiques d'un milieu, ainsi qu'à un microfermenteur correspondant. Ces propriétés optiques peuvent inclure notamment la turbidité, l'absorbance et la fluorescence du milieu, à une ou plusieurs longueurs d'onde ou sur des domaines spectraux prédéterminés.

Dans de nombreux laboratoires de biologie et également dans certaines industries, il est très souvent nécessaire de cultiver des micro-organismes en milieu liquide et de mesurer la concentration des cellules à différentes étapes de la culture. Cette quantification peut être faite de manière directe sur des échantillons, par comptage des cellules ou par mesure du poids sec. Quoique très précises, ces méthodes sont longues et fastidieuses et il est plus aisé de faire une évaluation de la quantité de cellules de manière indirecte, en mesurant par exemple la disparition de substrats ou l'apparition de composés intracellulaires (NADH ....), ou extracellulaires (alcools, acides organiques ....). Cependant, les méthodes les plus commodes et les plus utilisées pour évaluer les concentrations cellulaires de cultures reposent sur des propriétés optiques de ces dernières.

Quand la matière est frappée par des radiations électromagnétiques, celles-ci interagissent avec les charges électroniques des atomes: une partie de ces rayonnements traverse la matière sans changement de direction (rayonnement transmis) et l'autre est diffusée dans toutes les directions de l'espace, chaque particule éclairée se comportant alors comme une source lumineuse. La quantité de lumière diffusée par les particules présentes dans le milieu liquide augmente avec le nombre et la taille de ces particules; de plus, dans le cas où les particules sont des micro-organismes, il a été montré que la diffusion de la lumière ne se produit pas d'une manière homogène dans toutes les directions de l'espace mais en majorité dans une direction proche de celle du faisceau incident.

C'est en raison de ces phénomènes de diffusion que les cultures liquides de micro-organismes ont un aspect trouble, dont la densité augmente avec la concentration cellulaire. Les dispositifs qui permettent de quantifier ce trouble, ou turbidimètres, mesurent la turbidité de ces cultures.

Parmi les turbidimètres existant, on distingue les appareils de mesure en discontinu, avec lesquels on mesure de manière répétée la lumière transmise et/ou réfléchie par un milieu liquide, et les appareils de mesure en ligne, pour lesquels des sondes de mesure de turbidité sont introduites à l'intérieur d'un milieu liquide et reliées à un système d'enregistrement.

Les appareils de mesure en discontinu, tels que néphélomètres, colorimètres, spectrophotomètres et turbidimètres mixtes, ne permettent pas, pour la plupart d'entre eux, de mesurer des turbidités élevées. En effet, leur gamme de mesure ne s'étend habituellement pas au-delà de 1000 NTU (Nephelometric Turbidity Units) et est le plus souvent beaucoup plus limitée encore. Il est donc nécessaire de diluer des échantillons, ce qui augmente les risques d'erreur et la quantité de travail à fournir pour un expérimentateur En outre, la prise d'échantillons elle-même est très contraignante, car elle nécessite la présence d'un expérimentateur à intervalles réguliers pendant toute la durée d'une culture. Certains turbidimètres mixtes ont été conçus pour couvrir une plus large gamme de turbidité, mais il s'agit d'appareils coûteux.

Les turbidimètres en ligne, dont les gammes de mesure peuvent être assez vastes, ont pour inconvénients de nécessiter des volumes de culture important (1 litre environ) à cause de l'encombrement des sondes introduites à l'intérieur des cultures, ce qui limite leur utilisation au sein de fermenteurs. De plus, les cultures en fermenteurs sont généralement aérées et agitées, ce qui provoque de nombreuses bulles perturbant fortement les mesures de turbidité. Ces dispositifs sont aussi pénalisés par leur coût élevé.

La demande internationale de brevet WO-92/13.482 divulgue un appareil et une méthode de mesure d'un paramètre sanguin. L'appareil comprend une source de lumière rouge et une source de lumière infrarouge dirigeant de la lumière vers un échantillon sanguin et un détecteur recevant la lumière provenant des deux sources et renvoyée par le sang. Une boucle de rétroaction optique est utilisée pour chacune des deux sources de manière à ce que l'intensité de la lumière reçue par le détecteur soit approximativement constante pour une plage de valeur du paramètre sanguin, ce qui permet une commande précise des sources de lumière. Des courbes de référence permettent de déduire deux paramètres sanguins des signaux de rétroaction obtenus respectivement pour chacune des deux sources.

Un inconvénient de cette technique est qu'elle est limitée notamment par les capacités d'émission des sources de lumière et ne permet ainsi de couvrir qu'une plage restreinte de mesure avec une sensibilité suffisante. De plus, l'obtention de chaque résultat requiert un délai de stabilisation qui pénalise la rapidité des mesures. Une analyse en série d'un grand nombre d'échantillons ou des mesures sur des milieux hétérogènes et/ou perturbés par une circulation de bulles de gaz s'avère ainsi très difficile, ou même exclue. Par ailleurs, des imprécisions risquent d'être générées lors de l'exploitation des signaux de rétroaction, en raison de la dérive de valeurs capacitives.

Le brevet US-4.447.150 concerne un dispositif et une méthode de mesure de propriétés et de paramètres sanguins, utilisés pour mesurer le niveau de saturation d'oxygène sanguin. L'appareil divulgué comporte deux sources de lumière émettant respectivement à deux longueurs d'onde distinctes et un détecteur mesurant la lumière provenant de deux sources et renvoyée par du sang. La lumière réfléchie ou transmise par le sang est maintenue à un niveau constant au moyen d'une boucle de rétroaction optique. De plus, le rapport de deux tensions correspondant respectivement à des lumières mesurées en provenance des deux sources donne le pourcentage de saturation de l'oxygène sanguin.

Cette technique présente également l'inconvénient de ne pas permettre une large plage de mesures, ainsi que les autres inconvénients mentionnés pour le document WO-92/13.482.

La présente invention vise un appareil et un procédé de mesure n'ayant pas les inconvénients précédents, et permettant ainsi notamment d'effectuer des mesures dans différentes gammes de valeurs tout en maintenant une bonne sensibilité.

L'appareil et le procédé de l'invention peuvent aussi offrir une grande rapidité de mesure et une bonne précision.

En particulier, l'invention a pour objet un appareil de mesure de turbidité capable de mesurer, avec précision, la turbidité de cultures microbiennes sur une large gamme de valeurs, fiable, facile d'utilisation, aisément adaptable à des microfermenteurs et pouvant être peu encombrant et bon marché.

L'invention a aussi pour objet un appareil de mesure d'absorbance et un appareil de mesure de fluorescence, capables de mesurer avec les mêmes avantages précités l'absorbance moléculaire et/ou la fluorescence d'un milieu, et plus généralement, l'invention vise un appareil de mesure d'une ou plusieurs propriétés optiques d'un milieu, liquide ou solide.

L'invention concerne un tel appareil de mesure avantageusement automatisé, et permettant ainsi de réaliser des expériences de longues durées sans intervention d'un expérimentateur.

L'invention a également pour objet un tel appareil de mesure adaptable à des microfermenteurs en batterie, rendant possible d'effectuer des mesures simultanées pour les différents microfermenteurs.

L'invention est également relative à un microfermenteur muni d'un appareil de mesure ayant les qualités précédentes.

L'invention a notamment pour objet un microfermenteur ayant des systèmes d'alimentation en milieu de culture et en gaz qui sont simples et fiables, protégé de toute contamination extérieure et capable de recevoir des cultures de petits volumes. De même, le système protège l'extérieur de contaminations par la culture elle-même.

L'invention concerne également un microfermenteur à prélèvements automatisés pour certains stades de turbidité, d'absorbance, et/ou de fluorescence, prédéterminés et un microfermenteur à dilution automatique pour cultures cycliques.

L'invention a aussi pour objet un procédé de mesure de propriétés optiques pouvant inclure notamment la turbidité, l'absorbance et la fluorescence, rendant possible des mesures sur une large gamme de valeurs avec une grande précision, une bonne fiabilité et une bonne reproductibilité.

L'invention s'applique aux domaines de la microbiologie et de la biotechnologie, et plus généralement à toute mesure de composés troubles, par exemple pour l'analyse et le traitement d'eaux, d'effluents agricoles ou industriels ou de polluants (mesure de turbidité).

Elle s'applique aussi à des mesures *in vivo* ou *in vitro* de fluorescence, et plus précisément aux mesures de concentration de composés fluorescents intra ou extracellulaires.

A cet effet, l'invention a pour objet un appareil de mesure d'au moins une propriété optique d'un milieu, comprenant:

- une source de lumière destinée à émettre un faisceau lumineux d'intensité variable vers et dans le milieu,
- des moyens de détection destinés à mesurer l'intensité du faisceau renvoyé par le milieu liquide,
- des moyens de commande de l'intensité du faisceau émis par la source,
- des moyens de stockage destinés à stocker une valeur représentative d'une intensité nominale du faisceau renvoyé,
- un système de rétroaction relié aux moyens de commande, aux moyens de détection et aux moyens de stockage, agissant sur les moyens de commande de façon à ce que l'intensité mesurée du faisceau renvoyé soit égale à l'intensité nominale, et
- des moyens de lecture de l'intensité du faisceau émis, pour l'intensité nominale du faisceau renvoyé, ces intensités du faisceau émis et nominale du faisceau renvoyé étant représentatives de la ou des propriété(s) optique(s) du milieu.

Selon l'invention, l'appareil de mesure comprend des moyens d'ajustement de l'intensité nominale, reliés aux moyens de lecture et aux moyens de stockage, destinés à ajuster l'intensité nominale de façon à ce que l'intensité stabilisée du faisceau émis soit incluse dans une plage prédéterminée.

L'intensité nominale du faisceau renvoyé est ajustable. De cette manière, il est possible d'adapter l'appareil de mesure à une gamme donnée de valeurs de turbidité, d'absorbance et de fluorescence. Le choix de l'intensité nominale résulte d'un compromis. Elle doit être la plus élevée possible, de manière à ce que l'intensité du faisceau émis varie fortement avec la turbidité, l'absorbance et/ou la fluorescence, ce qui donne à l'appareil une très bonne précision de mesure. En revanche, l'intensité nominale doit être suffisamment faible pour pouvoir couvrir toute la gamme souhaitée sans que l'intensité du faisceau émis ne dépasse un seuil d'émission maximale de la source, provoquant sa saturation et/ou sa détérioration.

Cet appareil peut ainsi autoriser des mesures dans différentes gammes de valeurs, des plus faibles aux plus élevées, tout en gardant une sensibilité optimale. Dans l'état de la technique, au contraire, la plage de mesure est figée et est associée à la tension de référence utilisée.

Notamment, l'appareil rend possible des mesures de turbidité sur une large gamme de densités optiques avec une bonne précision. On obtient ainsi sans difficulté une gamme s'étendant de 45 à plus de 17.000 NTU, ce qui correspond pour une suspension de levure *Saccharomyces cerivisiae*, respectivement à 0,2 et plus de 75 unités de densité optique (DO) à 600 nm avec une erreur de 2% environ. Cet appareil offre aussi une bonne fiabilité et une bonne reproductibilité des mesures, correspondant à une faible dispersion des valeurs. Pour différents organismes, des courbes de correspondance entre turbidités et concentrations de cellules peuvent être faites aisément.

De plus, l'appareil de mesure selon l'invention peut être fabriqué à bas coût de réalisation, car il peut reposer sur l'emploi de composants électroniques peu nombreux et bon marché.

Les moyens de stockage sont avantageusement destinés à stocker une intensité de saturation et les moyens d'ajustement régulent l'intensité nominale à une valeur moindre lorsque l'intensité stabilisée du faisceau émis est supérieure à l'intensité de saturation.

Les valeurs pouvant être prises par l'intensité nominale sont avantageusement prédéterminées.

L'ajustement d'intensité nominale est ainsi automatisé, ce qui permet notamment de suivre la turbidité d'un milieu évolutif, sur une large gamme de turbidité et avec une très bonne précision. Il est particulièrement intéressant d'utiliser une diode luminescente, émettant préférentiellement dans l'infrarouge autour de 880 nm, pour cet ajustement automatisé. En effet, la tension appliquée aux bornes de la diode émettrice lors de l'émission du faisceau lumineux évolue proportionnellement avec la turbidité, et on obtient une excellente précision de mesure.

Le milieu mesuré peut être liquide ou solide (gels, préparations pour microscopie).

La ou les propriété(s) optique(s) mesurée(s) sont avantageusement choisies parmi la turbidité T, l'absorbance A ou la fluorescence F du milieu, pour un couple donné des spectres d'émission et de réception. Dans divers modes de réalisation, la lumière est émise et reçue selon respectivement les modalités suivantes:

- source de lumière monochromatique, ou à plus large bande, détection à une longueur d'onde correspondante (monochromatique ou à plus large bande),
- source de lumière monochromatique, ou à plus large bande, détection à une autre longueur d'onde (cas de fluorescence),
- source de lumière polychromatique, ou à plus larges bandes, détection à plusieurs longueurs d'onde correspondantes,
- source de lumière blanche, détection à une ou plusieurs longueurs d'onde sélectionnées par des filtres.

La détection de l'intensité du faisceau reçu à plusieurs longueurs d'onde permet d'obtenir des informations complémentaires sur le milieu.

La source de lumière est constituée d'un émetteur tel que par exemple diode, laser, couplés ou non à une fibre optique, destiné à émettre une lumière monochromatique ou à bandes spectrales, éventuellement de largeurs variables, ou blanche. L'émission peut être faite dans les domaines de l'UV, du visible, de l'infrarouge proche ou lointain.

Les moyens de détection consistent avantageusement en un ou plusieurs photodétecteur(s), tels que par exemple diode(s) (phototransistor, photodarlington) ou cellule(s) photoélectrique(s), ayant chacun une réception monochromatique ou avec une plus large bande passante, dans les domaines de l'UV, du visible, de l'infrarouge proche ou lointain. Dans les cas de mesures dans l'infrarouge, les moyens de détection sont avantageusement insensibles à la lumière visible, par commodité.

Par « faisceau renvoyé par le milieu », on entend un faisceau transmis sans interaction, diffusé, ou émis (dans les cas de fluorescence, par exemple).

Les moyens de détection sont placés à un ou plusieurs angle(s) approprié(s) par rapport à la direction du faisceau émis, préférentiellement à environ 180° (nécessairement à environ 180° en transmission).

Préférentiellement, le faisceau incident sur le milieu est parallèle. On obtient ainsi un très bon rapport lumière renvoyée/lumière émise.

Chaque longueur d'onde de mesure est choisie préférentiellement pour donner des informations sur un type distinct de particules.

Ainsi, pour des mesures de turbidité, deux longueurs d'onde distinctes permettent d'obtenir des résultats sur des particules de deux tailles différentes, coexistant en suspension. Il peut par exemple s'agir de cellules infectées par des particules phagiques et de phages libérés par les cellules lysées. En effet, pour un type de particules donné, la quantité de lumière diffusée ou transmise dépend de la longueur d'onde détectée. De plus, plus la longueur d'onde est élevée, plus le rapport lumière transmise/lumière émise est grand.

Une forme de réalisation pour mesurer aux deux longueurs d'onde consiste à émettre à l'aide de deux émetteurs de longueurs d'onde différentes l'une de l'autre, par exemple infrarouge et visible. Une autre forme de réalisation consiste à émettre en lumière blanche et à disposer des filtres sur un détecteur de manière à sélectionner les deux longueurs d'onde.

Pour des mesures d'absorbance, deux longueurs d'onde distinctes en réception permettent de quantifier deux types de molécules absorbant à des longueurs d'onde distinctes, ou à corréler des observations sur un type de particules.

De manière générale, des mesures à plusieurs longueurs d'ondes peuvent permettre de déterminer la proportion et la nature des particules, des cellules ou des molécules des milieux analysés, et de discriminer la taille, la forme, la concentration, la nature et/ou la proportion de différentes espèces cellulaires, particulaires et/ou moléculaires. Le nombre de longueurs d'onde étant égal à n, le traitement mathématique peut être réalisé grâce à un système à n équations et à n inconnues.

Avantageusement, l'intensité nominale (intensité fixée du faisceau renvoyé) est choisie en fonction de la gamme de concentration des particules à mesurer dans le milieu, l'intensité nominale diminuant quand la gamme de concentration à mesurer augmente.

L'appareil de mesure selon l'invention ne mesure pas simplement la lumière renvoyée par un milieu liquide pour déterminer la turbidité, l'absorbance ou la fluorescence, mais l'énergie fournie par la source permettant d'obtenir une quantité fixée de lumière renvoyée. Cette énergie, ou intensité du faisceau émis, donne la turbidité, l'absorbance ou la fluorescence du milieu liquide ou solide, pour chaque longueur d'onde de mesure, au moyen d'une relation mathématique simple établie grâce à des courbes d'étalonnage. Ainsi, lorsque la source de lumière émet une lumière infrarouge autour de 880 nm, la quantité d'énergie transmise par le milieu est inversement proportionnelle à la turbidité du milieu traversé et le système de rétroaction électronique ajuste automatiquement le faisceau émis pour que la lumière renvoyée soit égale à une valeur fixée.

Dans le cas de mesure de fluorescence, la quantité de lumière renvoyée est proportionnelle à l'intensité du faisceau émis et à la concentration de la substance fluorescente. De plus, elle est affectée par des phénomènes de filtre interne.

L'appareil de mesure permet aussi de s'affranchir de tous prélèvements autres que pour des analyses d'échantillon, ce qui facilite les manipulations, supprime des risques d'erreur au cours de dilutions subséquentes à des prélèvements et réduit les risques de contamination d'une culture ou de l'environnement (par exemple dans le cas de cultures de pathogènes).

Avantageusement, l'appareil de mesure peut être entièrement extérieur au milieu liquide. Ainsi, dans le cas où ce milieu comprend une culture, l'appareil n'a pas à être stérilisé et ne risque pas de se détériorer par encrassement ou recouvrement par des biofilms.

Préférentiellement, l'appareil de mesure est automatisé, notamment par un enregistrement automatique des données et par un ajustement automatique de l'intensité nominale du faisceau renvoyé. Ainsi, il est possible de réaliser des expériences de longue durée sans intervention d'un expérimentateur, notamment pour le suivi de cultures de jour comme de nuit sans la moindre surveillance humaine.

Un autre avantage de l'appareil de mesure est sa capacité d'adaptation à une grande variété de milieux liquides, et notamment à des cultures continues de tous types, telles que par exemple chemostat, turbitostat ou cultures cycliques.

Préférentiellement, la source de lumière et/ou les moyens de détection sont destinés à mesurer l'intensité du faisceau renvoyé autour d'au moins une longueur d'onde, la ou les propriété(s) optique(s) du milieu correspondant chacune à la, ou à l'une des, longueur(s) d'onde.

Dans un premier mode préféré de réalisation, la, ou les, propriété(s) optique(s) du milieu comprennent la turbidité du milieu.

Les moyens de détection sont alors disposés préférentiellement dans la direction du faisceau émis, en transmission, le rapport intensité reçue/intensité émise étant optimal. Dans une variante de réalisation, certains au moins des moyens de détection sont disposés en biais par rapport à la direction du faisceau émis, de façon à détecter de la lumière diffusée.

Dans le premier mode de réalisation, la source de lumière et/ou les moyens de détection sont préférentiellement destinés à des mesures en infrarouge, avantageusement autour de 880 nm.

Il est par exemple intéressant que la source de lumière soit une diode luminescente ou LED, émettant en infrarouge.

L'émission en lumière infrarouge avec des longueurs d'onde variant de 840 nm à 920 nm avec un pic de 880 nm simplifie la mise en oeuvre de l'appareil de mesure et donne une bonne précision. En effet, la diffusion de la lumière dépendant de la longueur d'onde, l'utilisation de lumière blanche, par exemple, risque d'être plus difficile à mettre en oeuvre et de donner des résultats moins précis qu'une lumière presque monochromatique en infrarouge. De plus, les rayonnements infrarouges traversent mieux la matière, ce qui est favorable pour la mesure de fortes turbidités. Une émission à d'autres longueurs d'onde ou en lumière blanche est cependant également possible.

Dans un deuxième mode préféré de réalisation, la ou les propriété(s) optique(s) du milieu comprennent l'absorbance du milieu, l'invention s'appliquant alors à la colorimétrie et à la spectrophotométrie sur du milieu liquide ou solide.

Dans un troisième mode préféré de réalisation, la, ou les, propriété(s) optique(s) du milieu comprennent la fluorescence du milieu.

Ainsi, la GFP (Green Fluorescence Protein) présente naturellement chez la méduse *Aequora victoria* émet par fluorescence, lorsqu'elle est excitée par des lumières UV ou bleue (2 pics d'excitation à 395 et 475 nm), une lumière verte, à différentes longueurs d'onde (509 et 540 nm), ce qui permet de la distinguer de la lumière incidente ou diffusée. L'émission ne nécessite la présence d'aucun substrat ou cofacteur. Il existe des versions modifiées du gène codant pour cette protéine ce qui a pour effet d'améliorer son rendement quantique et de modifier son spectre d'émission (HELM, R., Nature, vol. 373, pp. 663-664, 1995).

De même, il est possible de quantifier des composés intracellulaires ou extracellulaires émettant par fluorescence. Par exemple:

| Fluorophore | Longueur d'onde d'excitation | Longueur d'onde de fluorescence (émission) |
|---|---|---|
| NADH, NADPH | 350 | 450 |
| FAD | 450 | 520 |
| Amino-acide aromatiques | 275 | 310 |
| Tryptophane | 289 | 330-360 |
| Nucléotides | 280 | 375 |
| Riboflavines | 445 | 520 |
| Pyridoxine | 313 | 385-415 |
| Antibiotiques | variable | variable |
| F(420) | 425 | 472 |

Ainsi, le NADH, coenzyme d'oxydo-réduction, émet une fluorescence à 450 nm quand il est excité par une lumière de longueur d'onde de 350 nm (le NAD forme oxydée ne produit pas de fluorescence). Au cours de la croissance cellulaire, la quantité de NADH varie avec la concentration cellulaire (Li. J., Biotechnology and Bioengineering, vol.37, pp. 1043-1049, 1991).

Il en est de même pour différents colorants ou chromophores (appelés aussi fluorochromes ou fluorophores) fluorescents (fluoresceine, anticorps fluorescents, pigments, quinine ....).

L'introduction dans différentes cellules (procaryotes ou eucaryotes) des gènes codant pour la GFP et leur expression dans différentes conditions permet le monitorage en continu de la fluorescence. L'appareil de mesure selon l'invention permet aussi d'étudier des effets de filtre interne de milieux fluorescents.

Ce troisième mode de réalisation a des applications dans de nombreux domaines, comme la quantification de substances fluorescentes dans des gels, des « blots », des microplaques. De plus, des études *in vivo* sont possibles, comme la mesure d'expression de gènes par le suivi de fluorescence des protéines « tagées » pour lesquelles ils codent, permettant à la fois la quantification et la localisation cellulaires de ces dernières. Ces mesures faites en fermenteurs permettent en outre de faire des analyses en fonction du temps, de l'état physiologique des cellules et des conditions d'environnement de la culture.

De manière préférée, l'appareil de mesure comprend une unité de traitement reliée aux moyens de lecture et comportant les moyens de stockage, les moyens de stockage étant destinés à stocker au moins une équation déterminée par une courbe d'étalonnage donnant la ou les propriété(s) optique(s) du milieu en fonction de l'intensité du faisceau émis pour respectivement au moins une valeur de l'intensité nominale du faisceau renvoyé. L'unité de traitement extrait alors la ou les propriété(s) optique(s) du milieu à partir de l'intensité stabilisée du faisceau émis pour une des valeurs de l'intensité nominale, d'une part, et de l'équation correspondant à cette valeur de l'intensité nominale, d'autre part.

De cette façon, la détermination de la turbidité, de l'absorbance et/ou de la fluorescence du milieu à partir des mesures est automatisée, et peut être effectuée en temps réel.

Il est alors avantageux que l'unité de traitement comprenne une unité centrale et au moins une carte de conversion analogique/numérique et numérique/analogique, couplant l'unité centrale aux moyens de commande et aux moyens de régulation.

De telles cartes permettent de s'affranchir d'étages convertisseurs courant/tension et tension/courant.

De plus, l'unité de traitement extrait préférentiellement la propriété optique du milieu directement à partir d'une valeur proportionnelle à l'intensité du faisceau émis.

Cette réalisation contraste avec les techniques connues, dans lesquelles les propriétés optiques sont obtenues à partir de signaux de rétroaction, qui doivent passer par un système d'intégration pour être exploitables. Elle évite ainsi l'introduction d'imprécision sur les résultats.

Avantageusement, l'appareil de mesure comporte une horloge, des moyens d'enregistrement dans les moyens de stockage de valeurs représentatives de la, ou des, propriété(s) optique(s) du milieu, ainsi que des moyens d'activation répétée du système de rétroaction et des moyens d'enregistrement.

L'enregistrement des résultats est ainsi automatisé, les mesures étant avantageusement effectuées à intervalles réguliers afin de pouvoir suivre l'évolution de la propriété optique au cours du temps. Dans une première forme de réalisation, les valeurs enregistrées sont celles directement obtenues par la lecture de l'intensité du faisceau émis, ces résultats étant traités ultérieurement. Dans une seconde forme de réalisation, l'appareil de mesure détermine les propriétés optiques après chaque mesure, et ce sont elles qui sont enregistrées dans les moyens de stockage.

Préférentiellement, le système de régulation par rétroaction et les moyens de commande sont tels que l'égalisation de l'intensité mesurée et de l'intensité nominale du faisceau renvoyé est obtenue de manière continue.

Ainsi, le temps de réponse est négligeable et limité uniquement par les temps de réponse intrinsèques des composants électroniques, de l'ordre de la nanoseconde. Cette réalisation contraste avec les techniques connues, dans lesquelles l'ajustement se fait par essais successifs et rythmés par des impulsions périodiques. L'appareil de mesure peut de cette manière procurer une très grande rapidité de mesure, qui permet par exemple d'analyser en série un très grand nombre d'échantillons et de faire des mesures sur des milieux hétérogènes et/ou perturbés par une circulation de bulles de gaz. Dans ces derniers cas, l'appareil de mesure est muni de moyens pour effectuer des mesures en rafales, et pour faire une analyse statistique des valeurs recueillies au cours de chacune de ces rafales, afin d'en extraire des valeurs significatives.

L'invention a aussi pour objet un microfermenteur, muni d'un appareil de mesure selon l'invention.

Le fait que l'appareil de mesure puisse être entièrement extérieur au microfermenteur, qu'il ne nécessite pas de prélèvements et de dilutions correspondantes, et qu'il rende possible l'automatisation des mesures, permet de réaliser un microfermenteur disposant d'atouts considérables. Notamment, un petit volume de culture suffit pour les mesures, ce qui améliore la facilité d'utilisation. On bénéficie notamment d'un faible encombrement et d'une faible fréquence de changements des nourrices contenant les milieux de culture. De plus, le microfermenteur et ses systèmes d'alimentation et d'élimination d'effluents peuvent être complètement isolés du milieu extérieur. Leur mise en surpression assure simplicité et fiabilité, car l'utilisation de tuyaux de perte de charge connue permet d'obtenir des débits précis et d'éviter des pompes coûteuses et encombrantes. Elle offre une garantie de stérilité en empêchant toute contamination de la nourrice par la culture et de la culture par l'extérieur et permet la culture par exemple de souches pathogènes en toute sécurité.

D'autre part, l'automatisation des mesures rend possible l'automatisation de prélèvements pour des densités optiques correspondant à des états physiologiques précis, tels que par exemple, taux de croissance maximum, fin de croissance ou fin de phase stationnaire. Elle permet aussi d'effectuer des dilutions automatiques pour cultures cycliques.

Un autre avantage est que plusieurs microfermenteurs peuvent être associés en batterie, indépendamment ou en cascade, chacun contenant une culture dans des conditions bien définies et reproductibles. Les appareils de mesure associés aux différents microfermenteurs sont alors avantageusement reliés à un système central destiné à commander des mesures en parallèle.

Dans un mode de réalisation préféré, le microfermenteur comprend:

- un tube fermé par un bouchon, destiné à contenir une culture,
- une première canalisation, destinée à injecter de l'air stérile et du milieu de culture dans la culture, et
- une seconde canalisation, destinée à récupérer des effluents,

le tube et toutes les canalisations étant sous pression.

Dans une réalisation avantageuse pour cultures cycliques, le microfermenteur comprend un système de dilution destiné à diluer une culture. L'appareil de mesure comprend alors une horloge et une unité de traitement reliée au système de dilution, l'unité de traitement déclenchant cycliquement le système de dilution.

Ce dispositif est très avantageux pour faire évoluer des souches ou pour réaliser des études physiologiques sur des paramètres de croissance tels que, par exemple, taux et rendement de croissance ou mesure du temps de latence avant la reprise de la croissance.

Préférentiellement l'unité de traitement active de manière répétée le système de rétroaction, enregistre dans les moyens de stockage des valeurs représentatives de la, ou des, propriété(s) optique(s) du milieu, et déclenche le système de dilution lorsqu'elle constate sur une pluralité des dernières valeurs enregistrées une stagnation de la, ou des, propriété(s) optique(s) du milieu.

Dans des variantes de réalisation, l'unité de traitement déclenche d'autres actions sur le milieu, telles que par exemple l'apport de diverses solutions ou de gaz, lorsqu'elle constate la stagnation.

Dans un autre mode de réalisation, l'unité de traitement déclenche le système de dilution périodiquement, après une durée de culture prédéterminée.

L'invention concerne aussi tout système muni d'un appareil de mesure selon l'invention, tel que par exemple un fermenteur (adaptation par excroissance ou à l'intérieur), un appareil autonome ou un lecteur de microplaques.

Différents types de cultures sont par ailleurs utilisables: cultures discontinues (batch) éventuellement avec ajout continu ou séquentiel d'un ou plusieurs substrats (fed batch); cultures cycliques; turbidostat, permettant un maintien de la turbidité d'une culture à une valeur constante par ajout du milieu de culture à volume constant dans un bioréacteur, de façon que la turbidité oscille autour d'un point de consigne; ou chemostat, permettant un ajout de milieu de culture à volume constant dans un bioréacteur, de façon continue et à un débit inférieur au taux de croissance maximum du microorganisme.

Divers moyens d'introduction des fluides peuvent être utilisés, tels que par exemple pompes, électrovannes « tout ou rien » ou proportionnelles, débitmètres, plusieurs nourrices pour différents substrats ou composés, additionnés en fonction du temps ou de la densité optique et/ou plusieurs gaz.

De manière préférée, les canalisations sont raccordées au moyen de connecteurs comportant chacun une partie mâle reliée à une première des canalisations, une partie femelle reliée à une deuxième des canalisations, et des moyens de connexion de la partie mâle à la partie femelle, chacune de ces parties mâle et femelle comprenant:

- une extrémité allongée et creuse reliée à la canalisation correspondant à cette partie et communiquant avec cette canalisation, et
- un fourreau allongé, entourant cette extrémité et relié à cette canalisation.

L'extrémité de la partie mâle est introduite dans l'extrémité de la partie femelle, le fourreau de l'une des parties est introduit dans le fourreau de l'autre partie et les fourreaux sont fixés l'un à l'autre par les moyens de connexions.

Ainsi, les fourreaux peuvent assurer la protection des extrémités, dans lesquelles circule un fluide.

Préférentiellement, les connecteurs sont munis de bouchons qui assurent une fermeture étanche et sont stérilisés à la vapeur, par autoclave par exemple. Au cours de la connexion, les bouchons sont ôtés et les extrémités des fourreaux sont soumises à un bref contact avec une flamme, afin de conserver leur stérilité et celle de l'air qui est à proximité immédiate. Ce système permet de maintenir froides les zones internes des parties mâle et femelle et autorise ainsi une connexion immédiate sans détérioration de joints, tout en maintenant une parfaite stérilité.

L'étanchéité de la liaison entre les deux extrémités est avantageusement obtenue au moyen de joints respectivement solidaires des parties mâle et femelle et contre lesquels viennent respectivement buter les extrémités des parties femelle et mâle.

Préférentiellement, le fourreau de la partie mâle est introduit dans le fourreau de la partie femelle. La protection assurée par les fourreaux est ainsi encore plus sûre.

De plus, il est intéressant que les moyens de connexion comprennent une bague de serrage reliée à la canalisation correspondant au fourreau récepteur et un système de fixation (filetage, par exemple) prévu sur le fourreau introduit dans le fourreau récepteur et coopérant avec cette bague.

La bague de serrage est avantageusement usinée pour contenir une butée limitant le serrage. Cette réalisation évite une compression excessive des joints et donc leur détérioration.

Dans une autre forme de réalisation, des joints toriques sont adjoints aux zones internes des parties mâle et femelle. Ils permettent de renforcer la sécurité et l'étanchéité.

L'invention a aussi pour objet un procédé de mesure d'au moins une propriété optique d'un milieu, dans lequel:

- on émet un faisceau lumineux d'intensité variable vers ou dans le milieu, et

  - on mesure l'intensité du faisceau renvoyé par le milieu,
  - on ajuste l'intensité du faisceau émis de façon à ce que l'intensité du faisceau renvoyé soit égale à une intensité nominale fixée,
  - on lit l'intensité stabilisée du faisceau émis ainsi obtenue, et
  - on rapporte une valeur représentative de l'intensité stabilisée à une équation déterminée par une courbe donnant la, ou les, propriété(s) optique(s) en fonction de la valeur représentative de l'intensité stabilisée du faisceau émis pour l'intensité nominale du faisceau renvoyé, de façon à extraire la, ou les, propriété(s) optique(s) du milieu.

Selon l'invention, on ajuste l'intensité nominale de façon que l'intensité stabilisée du faisceau émis soit incluse dans une plage prédéterminée.

D'autres avantages et buts de l'invention apparaîtront à la lecture d'exemples donnés ci-après dans un but illustratif et non limitatif, en référence aux figures annexées sur lesquelles:

La Figure 1 est un schéma de principe d'un appareil de mesure selon l'invention.

La Figure 2 représente un microfermenteur équipé d'un appareil de mesure selon l'invention.

La Figure 3 schématise une station automatique comprenant huit microfermenteurs selon l'invention.

La Figure 4 représente une section longitudinale de la partie mâle d'un connecteur, avec bouchon, utilisé pour les microfermenteurs des figures 2 et 3.

La Figure 5 est une coupe transversale V-V de la partie mâle de la Figure 4.

La Figure 6 représente une section longitudinale de la partie femelle du connecteur correspondant à la partie mâle de la Figure 4, avec bouchon.

La Figure 7 est une coupe transversale VII-VII de la partie femelle de la Figure 6.

La Figure 8 montre le connecteur assemblé à partir des parties mâle et femelle des Figures 4 à 7.

La Figure 9 représente un schéma de réalisation électronique d'un appareil de mesure selon l'invention, applicable au microfermenteur de la Figure 2 et à la station automatique de la Figure 3.

La Figure 10 illustre un deuxième mode de réalisation électronique à la place de celui de la Figure 9.

La Figure 11 illustre un troisième mode de réalisation électronique à la place de ceux des Figures 9 et 10.

La Figure 12 représente un ensemble de courbes d'étalonnage préparées avec une culture de levure *Saccharomyces cerevisiae*, donnant la tension représentative de l'intensité du faisceau émis en fonction de la turbidité pour un ensemble de tensions représentatives de l'intensité nominale du faisceau transmis.

La Figure 13 montre des courbes de croissance, donnant l'évolution de la turbidité, en fonction du temps exprimé en heures, de trois souches de *Saccharomyces cerevisiae*, respectivement des types FY23, FY73 et FY1679, cultivées en milieu complexe YPD (extrait de levure, peptone, glucose) à 30°C, obtenues avec un appareil de mesure selon l'invention.

La Figure 14 montre en parallèle les courbes de croissance (Logarithme népérien de la turbidité en fonction du temps en heures) de la souche du type FY23 et du mutant du type FYBL2-5D/Dn049, cultivées en milieu complexe YPD à 30°C, obtenues avec un appareil de mesure selon l'invention.

La Figure 15 montre la courbe de croissance en culture cyclique automatisée de la souche du type. FY1679 cultivée en milieu complexe YPD à 30°C, obtenue avec un appareil de mesure selon l'invention.

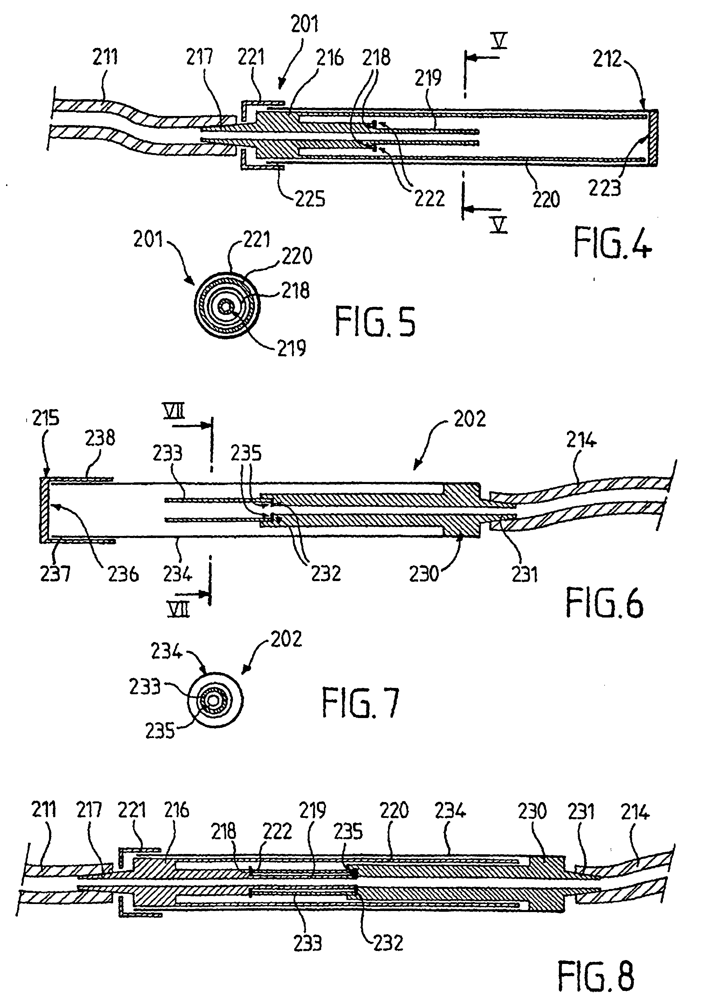

L'enseignement technique de ces figures, notamment les Figures 4 à 11 est considéré comme faisant partie intégrante de la description qui suit.

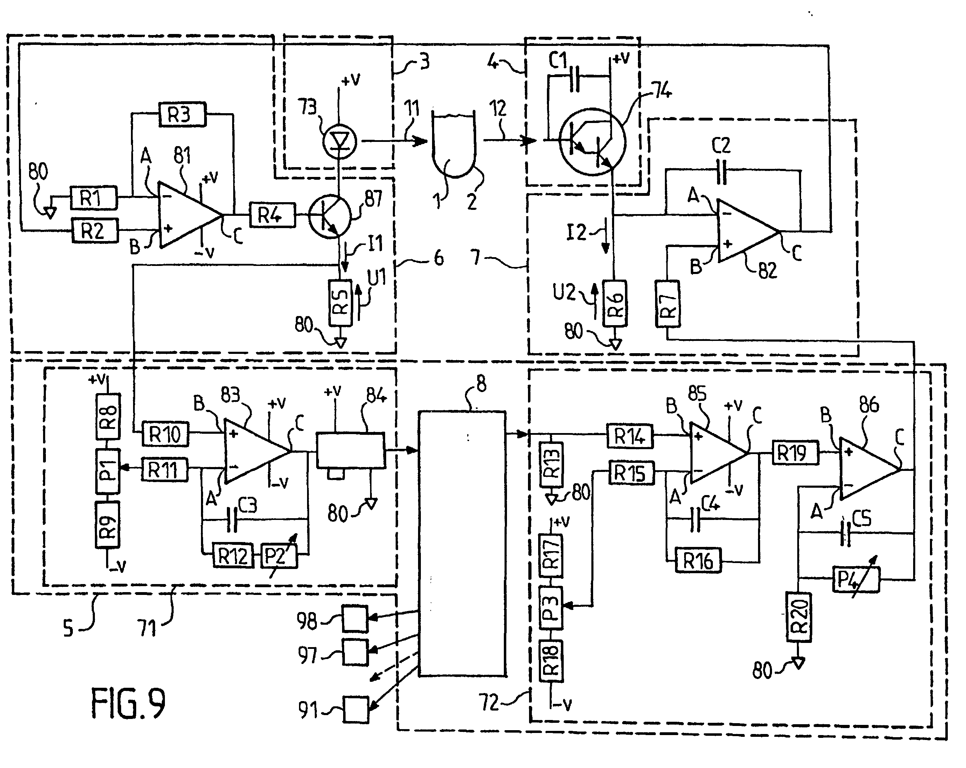

Sur les Figures 9 à 11, des éléments identiques sont identifiés par les mêmes références.

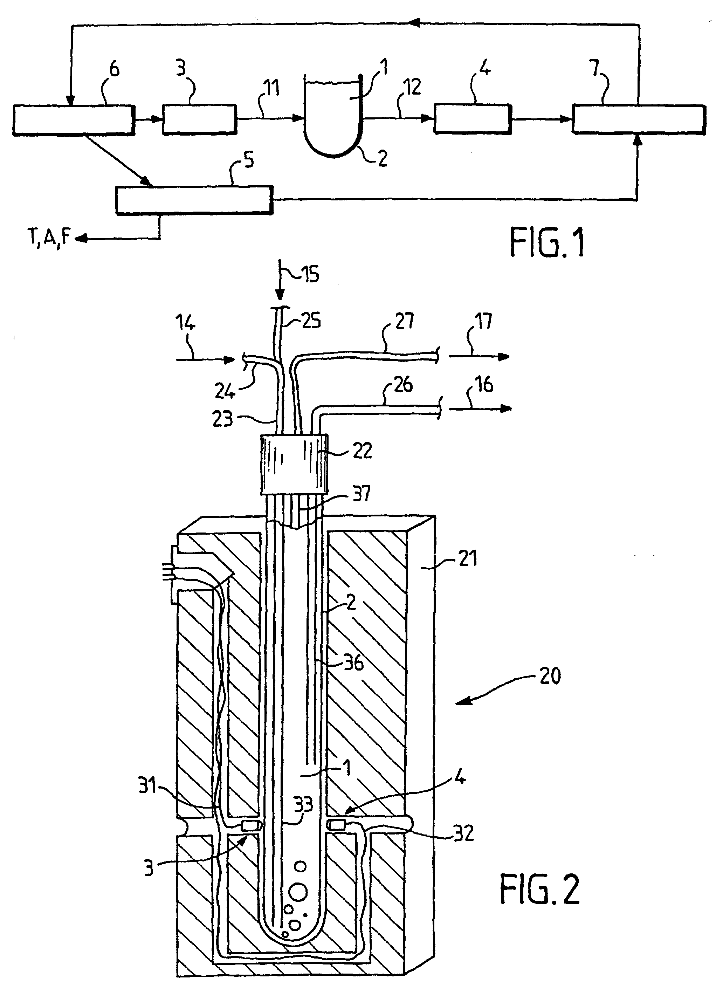

Un appareil de mesure de la turbidité T (Figure 1) d'un milieu liquide 1, contenue dans un récipient transparent 2, comprend un émetteur 3 qui émet un faisceau lumineux 11 vers le milieu 1. Préférentiellement, l'émetteur est presque monochromatique et il émet en infrarouge, par exemple autour de 880 nm.

Le faisceau 11 devient, après traversée du milieu 1, un faisceau transmis 12 dont l'intensité est mesurée par un photorécepteur 4. Le photorécepteur 4 est par exemple du type photodarlington. De manière préférée, l'émetteur 3, le récipient 2 et le photorécepteur 4 sont alignés. Dans des variantes de réalisation, le photorécepteur 4 n'est pas aligné avec l'émetteur 3 et le récipient 2, de telle sorte qu'il détecte un faisceau diffusé ou réfléchi. Le mode de réalisation préféré correspond cependant à une précision optimale.

L'émetteur 3 est relié à un bloc de commande 6, capable de lire et de modifier une tension U1, directement proportionnelle au courant I1 traversant l'émetteur 3 et par voie de conséquence l'intensité du faisceau émis 11. Le photorécepteur 4 est relié à une unité de régulation 7 destinée à imposer et à lire des valeurs représentatives du faisceau transmis 12. Préférentiellement, les valeurs représentatives du faisceau émis 11 et du faisceau transmis 12 sont linéairement proportionnelles aux courant I1 et I2 traversant respectivement l'émetteur 3 et le photorécepteur 4.

L'unité de régulation 7, reliée au photorécepteur 4 et à l'unité de traitement 5, est destinée à comparer une tension U2, directement proportionnelle au courant I2 traversant le photorécepteur 4, à une valeur de consigne V2. Cette dernière valeur, fournie par l'unité de traitement 5, est représentative d'une intensité nominale du faisceau transmis 12 pour une gamme de mesure donnée.

L'unité de traitement 5, outre des moyens de traitement des données, peut notamment comprendre des moyens permettant d'imposer la valeur de la tension V2 à l'unité 7, des moyens de lecture des valeurs représentatives U1 de l'intensité du faisceau émis 11, ainsi que des moyens de stockage des données. L'unité de régulation 7 est également reliée au bloc de commande 6 et agit sur celui-ci en fonction de l'écart entre les tensions U2 et V2. Cette boucle de rétroaction à laquelle concourent les unités 3, 4, 6 et 7, a ainsi pour fonction de réduire cet écart à zéro. A l'équilibre, la tension U2 est égale à la valeur de consigne V2 et la tension U1 est stabilisée à une valeur V1.

Cette tension V1 est représentative de la turbidité T du milieu 1. Cette tension V1 est envoyée à l'unité de traitement 5 qui, pour une valeur de consigne V2 donnée, extrait la turbidité T de la tension V1 grâce à une fonction mathématique établie à partir d'une courbe d'étalonnage de l'appareil de mesure donnant la turbidité T en fonction de la tension V1 pour la valeur V2.

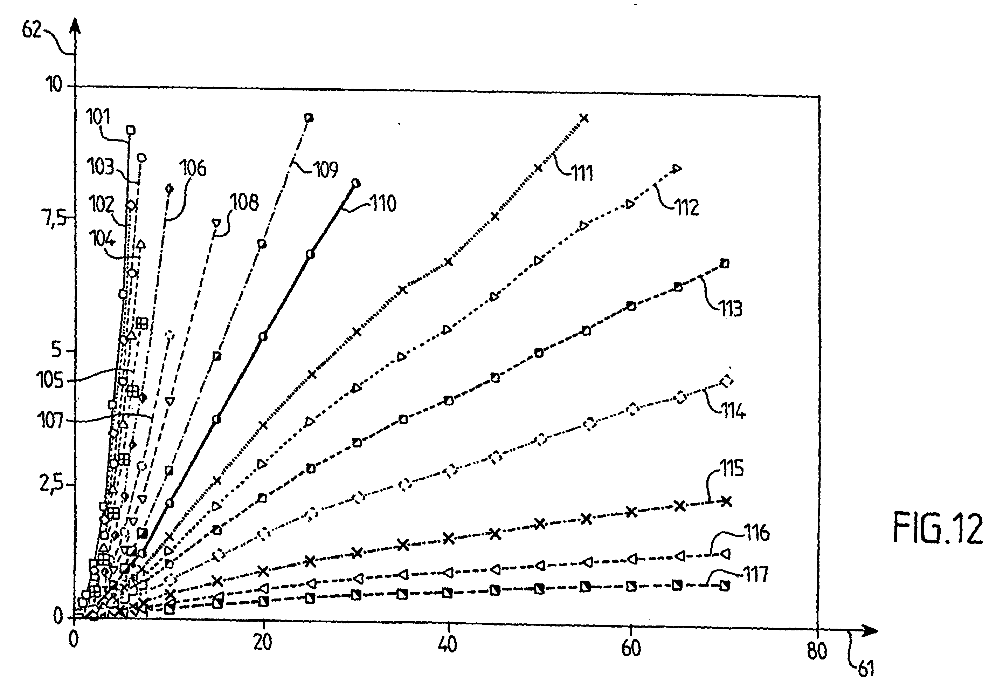

Un ensemble de courbes d'étalonnage 101-117 sont ainsi tracées sur la Figure 12 selon un premier axe 61 donnant la turbidité T en unité de DO et un second axe 62 donnant la tension stabilisée V1 en Volts. Ces courbes d'étalonnage 101-117 correspondent respectivement aux valeurs de consigne V2 suivantes:

8 V, 7 V, 6 V, 5 V, 4 V, 3 V, 2 V, 1,5 V, 1 V, 0,75 V, 0,5 V, 0,4 V, 0,3 V, 0,2 V, 0,1 V, 0,05 V, 0,025 V.

Le choix de la valeur de consigne V2 dépend de la gamme de turbidité T que l'on souhaite couvrir. En effet, plus la valeur de consigne V2 est élevée, plus la tension stabilisée V1 croît rapidement avec la turbidité T. Etant donné que la tension V1 est limitée supérieurement, la valeur V2 ne peut donc pas être maintenue pour un seuil supérieur correspondant à cette gamme de turbidité. Pour une gamme donnée, cependant, il est avantageux de choisir la valeur de consigne V2 la plus grande possible, afin d'obtenir la plus grande pente de variation de la tension V1 en fonction de la turbidité T, donc la meilleure précision de la mesure.

Préférentiellement, l'appareil de mesure ajuste automatiquement la valeur de consigne V2 en fonction des mesures. Pour ce faire, il procède avantageusement de la manière suivante. L'unité de traitement 5 dispose d'un seuil supérieur de la tension V1 et d'une liste de valeurs V2 prédéterminées et ordonnées de manière décroissante. Au début d'une série de mesures, l'unité de traitement 5 transmet à l'unité de régulation 7 la valeur V2 la plus élevée de la liste, puis la boucle de rétroaction ajuste la tension U1 afin que U2 soit égale à V2. La valeur V1 de la tension U1 est alors envoyée à l'unité de traitement 5, qui vérifie à chaque mesure que la tension stabilisée V1 reste inférieure à la tension de seuil prédéterminée. Si la tension V1 dépasse ce seuil, l'unité de traitement 5 envoie à l'unité de régulation 7 une valeur de consigne V2 plus faible et cette nouvelle valeur de consigne est adoptée pour les mesures suivantes. Dans le cas où une relation mathématique lie la valeur V1 à la valeur V2 pour plusieurs plages de turbidité, il est également possible de demander à l'unité de traitement 5 de calculer elle-même les valeurs de consigne V2 les mieux appropriées pour le calcul de la turbidité.

Préférentiellement, l'appareil de mesure détermine automatiquement après chaque mesure la turbidité T. Cette détermination est alors effectuée par l'unité de traitement 5 à partir des courbes d'étalonnage 101-117.

L'appareil de mesure est avantageusement appliqué à un microfermenteur 20, comme représenté sur la Figure 2. Dans l'exemple de réalisation présenté, le récipient 2 est un tube de verre, par exemple de section carrée de 20 mm de côté et de 160 mm de hauteur, ce qui donne un volume de culture utile d'environ 55 à 60 ml. Plus généralement, le tube de verre 2 contient un volume utile avantageusement compris entre 10 et 60 ml. Le tube 2 est logé dans un bloc 21 en alliage métallique, lui-même posé sur un bain sec thermostaté. Le bloc lui-même peut être équipé d'un système de chauffage autonome. Le tube 2 est surmonté d'un bouchon 22, à travers lequel passent plusieurs petites canalisations en verre ou en matière plastique. Une première de ces canalisations en verre, référencée 33, arrive au fond du tube 2, injectant constamment de l'air ou du gaz stérile 14 dans la culture. Elle peut aussi, à certains moments, injecter du milieu de culture neuf 15. Cette canalisation en verre 33 est reliée, extérieurement au tube 2 à une canalisation d'alimentation 23 dans laquelle convergent les canalisations d'alimentation 24 et 25, respectivement en air ou gaz stérile 14 et en milieu de culture 15. Les canalisations 24 et 25 sont préférentiellement équipées d'une électrovanne. Le microfermenteur 20 comprend aussi une deuxième canalisation en verre 36 qui débouche à une hauteur médiane du tube 2 et qui permet de réaliser l'inoculation et les prélèvements. La canalisation en verre 36 est reliée, extérieurement au tube 2, à une canalisation d'extraction 26 pour prise d'échantillon 16.

Le microfermenteur 20 comprend également une troisième canalisation en verre 37, placée en haut du tube 2, reliée à une bonbonne d'effluents par l'intermédiaire d'une canalisation d'élimination 27, extérieure au tube 2, qui permet d'éliminer des effluents 17.

Les canalisations extérieures 23-27 sont préférentiellement souples.

L'émetteur 3 et le photorécepteur 4 sont insérés dans des ouvertures du bloc 21 en alliage métallique, de part et d'autre du tube 2, et sont reliés par l'intermédiaire respectivement de fils électriques 31 et 32 au système de mesure.

L'ensemble du microfermenteur 20, avec ses canalisations extérieures 23-27, ainsi que la nourrice fournissant le milieu de culture 15 au microfermenteur 20, sont de préférence en surpression. De plus, l'air ou le gaz stériles 14 ne circule que dans un sens, ce qui empêche toute contamination. Les canalisations extérieures 24 et 25 sont alors avantageusement des tuyaux de perte de charge connue, permettant d'obtenir des débits précis. L'ensemble du montage est stérilisable à la chaleur et totalement étanche.

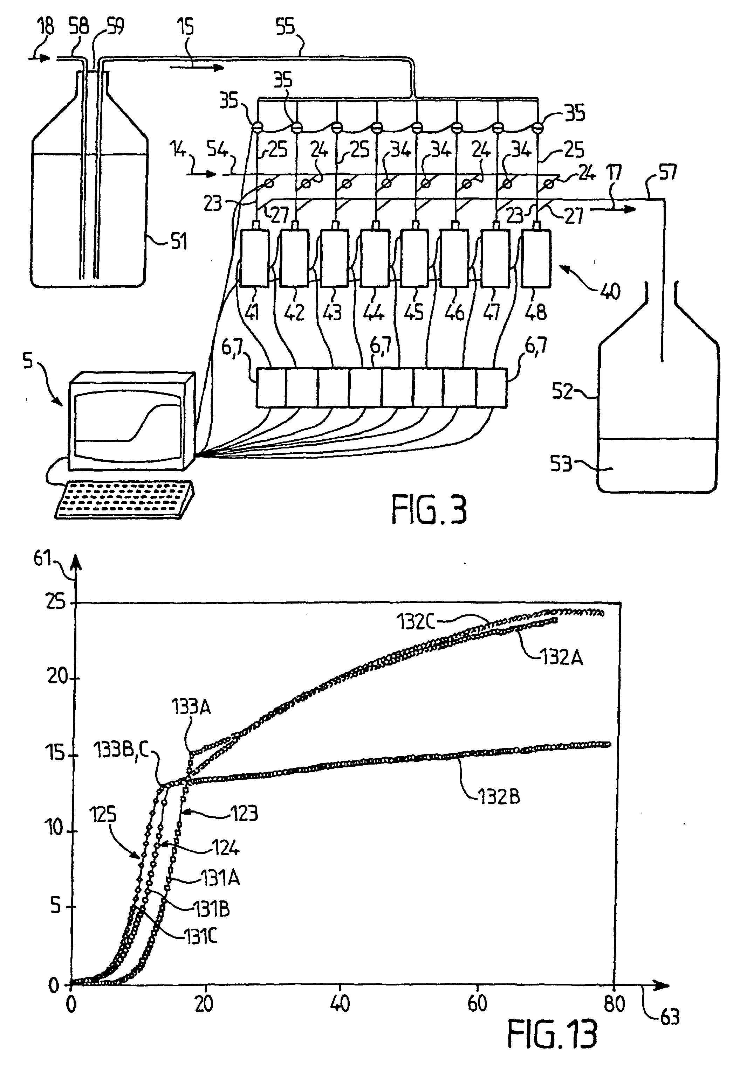

Dans un premier mode de réalisation, l'appareil de mesure de turbidité est appliqué à un microfermenteur isolé. Dans un second mode de réalisation, illustré sur la Figure 3, un ensemble d'appareils de mesure de turbidité sont appliqués à une batterie 40 de 8 microfermenteurs. A titre d'exemple, la batterie 40 comprend 8 microfermenteurs 41-48 du type du microfermenteur 20 décrit plus haut. L'ensemble des canalisations extérieures 24, 25 et 27 des microfermenteurs 41-48 sont alors respectivement couplées à trois canalisations centrales 54, 55 et 57. Chacune des canalisations 25 est équipée d'une électrovanne 35. De même, chacune des canalisations 24 est équipée d'une électrovanne 34.

Les canalisations 27 peuvent être équipées d'électrovannes pour permettre des prélèvements automatiques. La canalisation 57, sur laquelle convergent les canalisations 27, débouche dans une bonbonne 52 d'effluents destinée à contenir des déchets 53.

La nourrice du milieu de culture 15, sous pression, comprend un récipient 51 clos par un bouchon 59 et alimenté en air ou gaz stérile 18 par une canalisation 58 traversant le bouchon 59 et débouchant au fond du récipient 51. La canalisation 55 traverse le bouchon 59 et arrive au fond du récipient 51. Ce dispositif permet d'ajuster la pression régnant au fond de la nourrice. Cette pression est égale à la pression du gaz de la canalisation 58, quelle que soit la hauteur de liquide (vase de Mariotte).

Les canalisations 23-27, 33, 36, 37, 54, 55 et 57 sont préférentiellement raccordées au moyen de connecteurs 200, représentés sur la Figure 8 et détaillés ci-après.

Le connecteur 200 comprend:

- une partie mâle 201 (Figures 4 et 5) reliée à un premier tuyau 211 constituant une des canalisations et fermée par un premier bouchon 212, et
- une partie femelle 202 (Figures 6 et 7) reliée à un second tuyau 214 constituant une autre des canalisations et fermée par un second bouchon 215.

La partie mâle 201 comporte:

- un corps 216 préférentiellement en métal inoxydable, branché au tuyau 211 par une partie pouvant être légèrement effilée 217,
- un rebord interne 218,
- une extrémité 219 interne,
- un fourreau 220,
- un système de serrage à pas de vis 221 sous forme de bague, et
- un joint 222 plat et rond percé en son centre.

Le bouchon 212 de la partie mâle 201 a un fond muni d'un joint 223 plat et rond, et une extrémité 225 pourvue d'un filetage extérieur qui lui permet de se visser avec le système de serrage 221.

La partie femelle 202 comporte:

- un corps 230 préférentiellement en métal inoxydable, branché au tuyau 214 par une partie pouvant être légèrement effilée 231,
- un rebord interne 232,
- une extrémité 233 interne,
- un fourreau 234 ayant une extrémité 237, pourvue d'un filetage extérieur, et
- un joint 235 plat et rond percé en son centre.

Le bouchon 215 de la partie femelle 202 a un fond muni d'un joint plat 236 et une extrémité 238 pourvue d'un filetage intérieur qui lui permet de se visser sur l'extrémité 237 externe du fourreau 234.

Avant la connexion, les parties mâle 201 et femelle 202, branchées respectivement aux tuyaux 211 et 214 et munies de leurs bouchons 212 et 215 respectifs, sont stérilisés, par exemple à la vapeur.

La connexion s'effectue de la manière suivante:

- on dévisse les bouchons 212 et 215 des parties mâle 201 et femelle 202 sans les ôter;
- puis, en se plaçant au-dessus d'une flamme, on oriente vers le bas les extrémités 219 et 233 des parties mâle 201 et femelle 202; cela a pour conséquence de faire tomber les deux bouchons 212 et 215;
- on passe ensuite rapidement les fourreaux 220 et 234 à la flamme;
- on introduit la partie mâle 201 dans la partie femelle 202 jusqu'à ce que l'extrémité 219 soit en contact avec le joint 235 et que l'extrémité 233 soit au contact avec le joint 222; et
- on visse le système de serrage 221 sur le filetage de l'extrémité 238 du fourreau 234.

On obtient ainsi le connecteur 200, offrant une connexion étanche et fiable, avec protection par les fourreaux 220 et 234 comme représenté sur la Figure 8.

Les émetteurs 3, photorécepteurs 4, unité de commande 6, et de régulation 7 ainsi que les électrovannes 34 et 35, associés respectivement aux microfermenteurs 41-48 sont reliés à une ùnité de traitement 5 qui contient une unité centrale 8 composée d'un micro-ordinateur couplé à un système d'interfaçage analogique/numérique et numérique/analogique.

Un premier schéma de réalisation électronique particulier d'un appareil de mesure de turbidité, représenté sur la Figure 9, est applicable à un microfermenteur isolé ou à un microfermenteur d'une batterie.

Dans le schéma électronique, la masse est désignée par la référence 80 et les amplificateurs opérationnels ont leurs bornes d'entrée négative et positive et leur borne de sortie respectivement référencées A, B et C.

L'alimentation, symétrique, est préférentiellement stabilisée et régulée, à une tension +V égale à + 12 Volts et - V égale à -12 Volts.

De plus, toutes les résistances fixes sont à titre d'exemple à couche métallique 1/4 w et les condensateurs sont non polarisés. On donne par ailleurs les valeurs des résistances ajustables et des potentiomètres pour 10 tours - 1/2 W.

L'émetteur 3 comprend une diode électroluminescente 73 ou LED à infrarouge, et le photorécepteur 4 comprend un phototransistor 74 du type Darlington. Ce phototransistor 74 est muni d'un condensateur C1 de 0,1 µF, servant à éviter les oscillations.

Le bloc de commande 6 comprend un amplificateur opérationnel 81, dont l'entrée positive B reçoit une tension commandée et dont l'entrée négative A est reliée à la masse 80 par la résistance R1. La sortie C de l'amplificateur 81 commande, par le biais d'un transistor 87 jouant le rôle d'organe tampon ou buffer, l'intensité de courant I1 traversant la diode 73, et par conséquent l'intensité du faisceau 11 émis. Le gain de l'amplificateur 81 est fixé à 2 par des résistances R1 et R3 de 1 kΩ chacune. Le bloc de commande 6 comprend également une résistance R5 de 90,9 Ω, aux bornes de laquelle on mesure la tension U1. Cette résistance R5 remplit une fonction de conversion courant/tension et limite le courant maximal traversant la diode 73 à 100mA.

Le bloc de commande 6 comprend également deux résistances R2 et R4 valant respectivement 1 kΩ et 100 Ω.

L'unité de régulation par rétroaction 7 comprend une résistance R6 de 1 kΩ, qui transforme l'intensité 12 sortant du phototransistor 74 en la tension U2. L'unité de régulation par rétroaction 7 comprend également un amplificateur opérationnel 82, dont l'entrée négative A reçoit cette tension U2, dont l'entrée positive B reçoit la valeur de consigne V2 et dont la sortie C est reliée à l'entrée positive B de l'amplificateur 81 du bloc de commande 6. Le gain de l'amplificateur 82 est très grand du fait qu'il n'y a pas de résistance de contre-réaction, d'où une grande sensibilité aux variations de l'intensité du faisceau transmis 12, entraînant une grande efficacité du rétrocontrôle. L'amplificateur 82 est muni d'un condensateur C2 de 1 µF, qui sert à éviter des oscillations.

L'unité de régulation 7 comporte aussi une résistance R7, reliée à l'entrée positive B de l'amplificateur 82, valant 1 kΩ.

Dans l'exemple illustré, l'unité de traitement 5 est constitué, d'une part, d'un étage 72 convertisseur courant/tension qui le relie à l'unité 7, d'un étage 71 convertisseur tension/courant qui le relie à l'unité 6 et d'autre part, d'une unité centrale 8 constituée d'un micro-ordinateur muni d'une interface de conversion analogique/numérique, numérique/analogique communiquant par boucle de courant 4-20 mA. Ce dernier type de communication par boucle de courant rend les étages 71 et 72 nécessaires. Un système informatique muni d'une carte de conversion travaillant directement en tension permet de se passer des étages 71 et 72.

L'étage convertisseur 71 repose sur l'utilisation d'un amplificateur opérationnel 83, dont l'entrée positive B reçoit la tension U1, et sur un circuit convertisseur 84 tension/courant, dont l'entrée est reliée à la sortie C de l'amplificateur 83. L'étage convertisseur 71 comporte également des résistances R8-R12 valant respectivement 2,43 kΩ, 2,43 kΩ, 1 kΩ, 1 kΩ et 2,25 kΩ. Il comporte aussi un potentiomètre P1 de 200 Ω, une résistance ajustable P2 de 50 Ω et un condensateur C3 de 1 µF.

L'étage convertisseur 72 repose sur l'utilisation de deux amplificateurs opérationnels 85 et 86. L'entrée positive B de l'amplificateur 85 reçoit une tension de l'unité centrale 8, représentative de la valeur de consigne V2. La sortie C de l'amplificateur 85 est reliée à l'entrée positive B de l'amplificateur 86 et la sortie C de l'amplificateur 86 est reliée à l'entrée positive B de l'amplificateur 82 de l'unité de régulation par rétroaction 7. Ainsi, l'unité de traitement 5 envoie la valeur de consigne V2 à l'unité de régulation par rétroaction 7, ce qui lui permet, le cas échéant, de donner un ordre de changement de gamme de mesure.

L'étage convertisseur 72 comporte également des résistances R13-R20, les résistances R14-R16, R19 et R20 valant 1 kΩ, et les résistances R13, R17 et R18 valant respectivement 250 Ω, 1,3 kΩ, et 1,82 kΩ. L'étage convertisseur 72 comprend aussi un potentiomètre P3 de 500 Ω, une résistance ajustable P4 de 1 kΩ et des condensateurs C4 et C5 de 1 µF chacun.

L'unité de traitement 5 comporte avantageusement une unité de commande, destinée à commander l'ouverture et la fermeture d'électrovannes en nombre variable disposées sur les circuits d'arrivée du milieu de culture 15 et aussi d'air ou de gaz stériles 14. A titre d'exemple, l'unité de traitement 5 agit sur les électrovannes 34 et 35 des microfermenteurs 41-48 par l'intermédiaire respectivement de systèmes de commande 91-98.

En guise d'exemple de réalisation, la diode 73 et le phototransistor 74 dont le constructeur est la société Honeywell, sont du type respectivement commercialisé sous les noms SE5470-003 et SD5410-002 et les amplificateurs opérationnels 81, 82, 83, 85, 86 sont fabriqués et commercialisés, sous le nom LM358A par la société National Semi Conducteurs (NSC). Le circuit convertisseur 84 est du type 2B20A et est fabriqué par la société Analog Devices (AD) tandis que le transistor 87 est commercialisé sous la référence 2N2222.

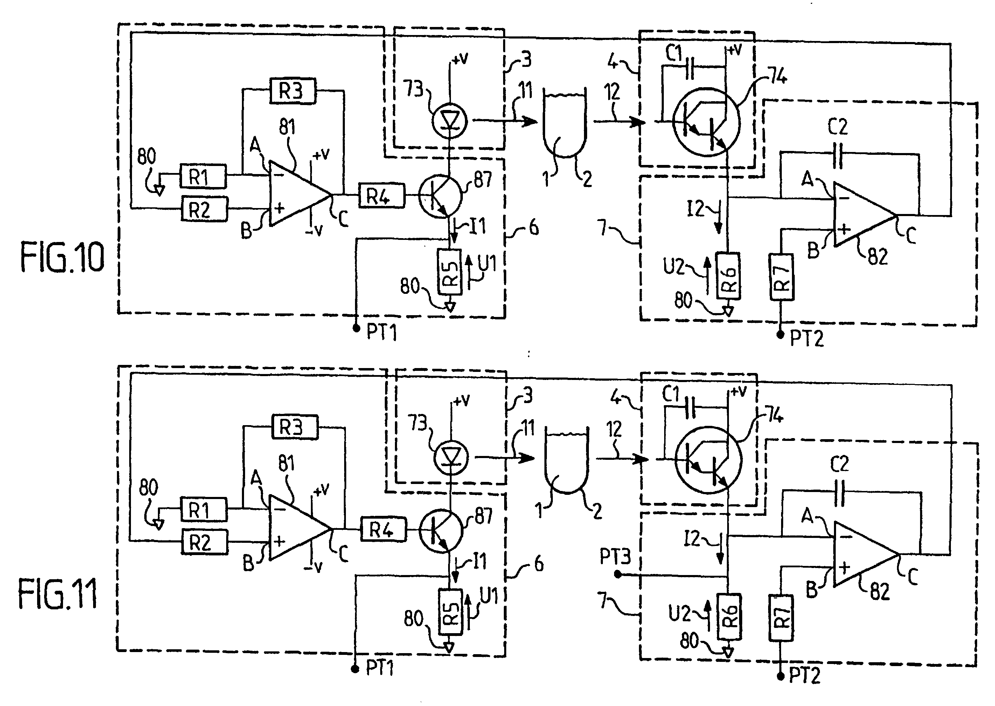

Selon un deuxième schéma de réalisation électronique (Figure 10), on s'affranchit des étages 71 et 72 au moyen d'une carte électronique, qui effectue directement les conversions analogique/numérique et numérique/analogique et communique les résultats et les ordres sous forme numérique via un bus de communication.

La tension U1 aux bornes de la résistance R5 est alors transmise à la carte sous forme de signal analogique par un point PT1 de sortie. De plus, l'entrée positive B de l'amplificateur opérationnel 82 est reliée à la carte par l'intermédiaire de la résistance R7, par un point PT2 d'entrée. La carte communique ainsi sous forme analogique à l'unité de régulation 7 la valeur de consigne V2.

Selon un troisième schéma de réalisation électronique (Figure 11), variante du deuxième schéma, l'entrée négative A de l'amplificateur opérationnel 82 est également reliée à la carte, par un point PT3 de contrôle. Ce point PT3, qui permet de transmettre la valeur de la tension U2, est destiné à vérifier le bon fonctionnement de l'ajustement de tension et de la rétroaction. Il rend ainsi possible, par repérage des différences entre les tensions U2 et V2, un contrôle global de tous les éléments de la chaîne.

Préférentiellement, tous les composants du bloc de commande 6 et de l'unité de régulation 7 sont implantés sur une carte électronique.

Avantageusement, plusieurs cartes sont intégrées dans un boîtier, dont une carte d'initialisation spécifique.

En fonctionnement, il est intéressant de procéder de la manière décrite ci-après pour l'un quelconque des microfermenteurs 41-48 de la batterie 40, les microfermenteurs étant indépendants les uns des autres car pilotés par un programme informatique distinct. A intervalles réguliers, un compteur de temps se met en route et quand le temps est égal à une valeur fixée, les événements suivants sont déclenchés:

- Un signal est envoyé par le programme de l'unité centrale 8 qui déclenche ainsi, par le biais d'un relais (systèmes de commande 91-98), l'électrovanne 34; celle-ci interrompt le passage de l'air ou du gaz stériles dans les microfermenteurs 41-48 afin de ne plus provoquer de bulles, source d'erreurs dans les mesures.
- Grâce au programme, la valeur de consigne V2 est injectée dans l'unité de régulation par rétroaction 7.
- La valeur de consigne V2 étant fixée, l'unité de régulation par rétroaction 7, fonctionnant de manière autonome, ajuste la tension U1 pour émettre la quantité exacte de lumière qui, après passage à travers le tube 2, provoque l'apparition d'une tension U2 égale à la valeur de consigne V2 et proportionnelle au courant 12 traversant le photorécepteur 4.
- La valeur de la tension U1 est convertie en valeur digitale et enregistrée par le programme.
- Le couple émetteur 3 - photorécepteur 4 est mis en repos.
- L'arrêt de l'électrovanne 34 rétablit l'aération ou le passage de gaz.
- Le compteur de temps est remis à zéro.
- Si la valeur de U1 mesurée ne dépasse pas certains seuils fixés, le programme calcule la valeur de turbidité T correspondante, en utilisant une équation déterminée antérieurement de, manière empirique. A chaque valeur de consigne V2, correspond une équation par exemple de la forme:

$$T = a \times U1^3 + b \times U1^2 + c \times U1 + d,$$

où les coefficients a, b, c et d ont été déterminés expérimentalement pour la valeur de V2.

- Le cycle recommence.
- Si la valeur de U1 mesurée dépasse le seuil fixé, alors une valeur de consigne V2 plus basse est choisie et le compteur de temps est incrémenté artificiellement afin de refaire aussitôt une mesure avec ce nouveau paramètre, puis la turbidité T est calculée.
- Le cycle recommence.

Les valeurs de la turbidité T sont affichées sur l'écran du micro-ordinateur de l'unité centrale 8, ce qui permet de suivre l'évolution de la culture au cours du temps. De plus, ces valeurs sont archivées dans un fichier, afin de traiter les données ultérieurement, par exemple avec un tableur.

Dans une variante de réalisation et de mise en oeuvre, on effectue des mesures en rafales. L'utilisation de l'électrovanne 34 devient alors inutile.

Il est intéressant que le dispositif précédemment décrit soit utilisé pour automatiser les cultures cycliques. Dans une mise en oeuvre avantageuse, un algorithme de contrôle détermine l'accroissement de population des cellules en faisant un calcul de régression linéaire sur les n dernières valeurs de turbidité T mesurées, n pouvant être réglable. La pente de la droite de régression obtenue est calculée: si elle est inférieure ou égale à une valeur fixée, l'accroissement de population est considéré comme nul et un compteur de temps se met en route. Si la culture se remet à croître, par exemple après une phase de diauxie, le compteur est remis à zéro. Quand le compteur atteint une valeur fixée, le programme de l'unité centrale 8 déclenche l'ouverture de l'électrovanne 35 concernée, via les interfaces, ce qui permet une alimentation en milieu de culture frais pendant un temps déterminé, afin de faire une dilution exponentielle. Puis, les cycles de mesure recommencent.

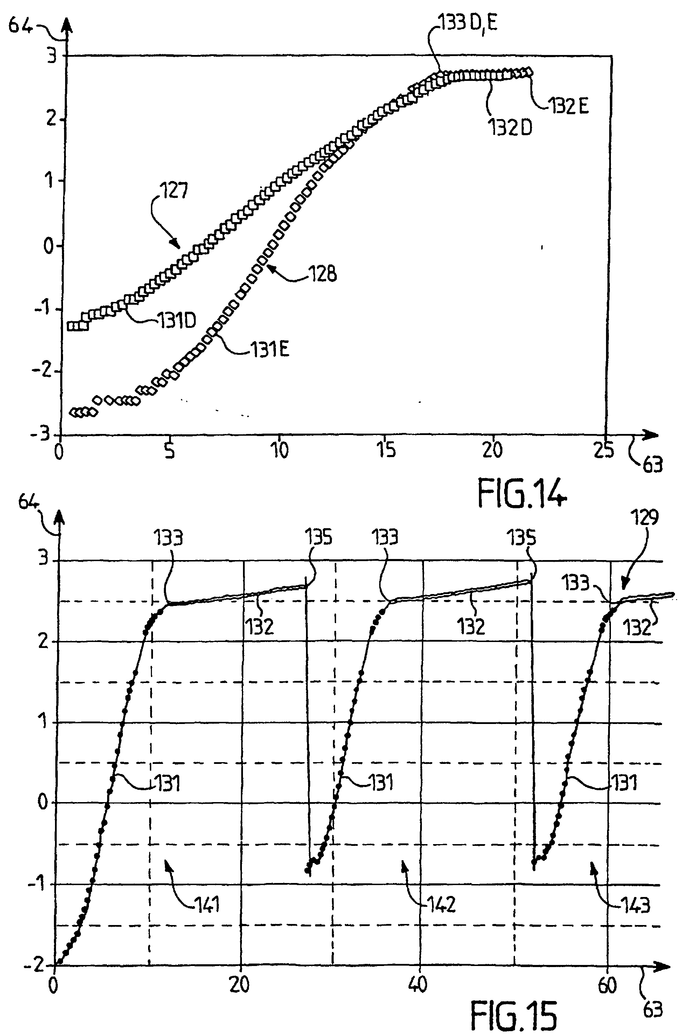

Nous exposons ci-après divers résultats obtenus au moyen d'un microfermenteur équipé d'un appareil de mesure de turbidité conforme à la description précédente. Des courbes de croissance ont été obtenues à partir de souches de levure *Saccharomyces cerevisiae* cultivées en milieu complexe YPD à 30°C. Les résultats obtenus, représentés sur les Figures 13 à 15, montrent une grande homogénéité des valeurs, c'est-à-dire très peu de dispersion, et des différences de l'ordre de 2% par rapport à des mesures effectuées en parallèle avec un spectrophotomètre, sur des prélèvements de ces cultures. En raison de sa fiabilité, l'appareil de mesure de turbidité permet donc de comparer très aisément les croissances des souches.

De manière générale, les courbes de croissances, qui donnent la turbidité T en fonction du temps, comportent chacune une partie 131 avec un taux élevé (pente forte) de croissance suivie d'une partie 132 à taux de croissance faible (pente faible), séparées par un plateau 133. Ainsi, les courbes de culture des souches sauvages FY23, FY73 et FY1679, référencées respectivement 123, 124 et 125 et tracées sur la Figure 13 selon un axe 63 de temps (en heures) et l'axe 61 de turbidité T (en unités de DO), ont respectivement des parties à forts taux de croissance 131A, 131B et 131C, des parties à faibles taux de croissance 132A, 132B et 132C et des plateaux de transition 133A, 133B et 133C.

La comparaison de ces courbes de croissance 123-125 montre que la souche FY73, après avoir consommé tout le glucose du milieu, pousse très lentement sur l'éthanol produit par fermentation du glucose. En revanche, les souches FY23 et FY1679 ont une croissance plus rapide sur éthanol (parties à pentes faibles 132A et 132C). De plus, les plateaux 133B et 133C de diauxie des souches FY73 et FY1679 sont initiés à une turbidité plus basse (à 12,8 unités de DO) que le plateau 133A de la souche FY23 (à 14,8 unités de DO).

D'autres résultats, représentés sur la Figure 14 selon l'axe 63 de temps en heures et un axe 64 donnant le logarithme népérien de la turbidité T (lnDO), représentent les courbes de croissance 127 et 128, respectivement, du mutant FYBL2-5D/Dn049 et de la souche FY23. Les courbes 127 et 128 comportent respectivement des parties à fort taux de croissance 131D et 131E, des parties à faible taux de croissance 132D et 132E et des plateaux de transition 133D et 133E. On constate que la souche FY23 a un taux de croissance égal à 0,545 h$^{-1}$, qui est presque le double du taux de croissance de la souche FYBL2-5D/Dn049, égal 0,286 h$^{-1}$.

Une courbe de croissance 129 d'une culture cyclique de FY1679 dont les dilutions sont complètement pilotées par micro-ordinateur, représentée sur la Figure 15, comporte une succession de cycles 141-143 similaires. Chacun de ces cycles inclut une partie à fort taux de croissance 131 et une partie à faible taux de croissance 132, séparées par un plateau de transition 133. La dilution est déclenchée en un point de dilution 135, 24 heures après le début de la culture. La dilution peut être également déclenchée lorsque le taux de croissance est devenu quasi-nul, ou après une certaine durée de phase stationnaire de la culture.

Dans un autre mode de réalisation, l'appareil de mesure comprend:

- un couple émetteur/récepteur de lumière intégré à l'appareil,
- un dispositif d'interfaçage comportant un bus de circulation de données entre un dispositif de traitement automatisé des données et un ou plusieurs boîtiers de mesure, et de relais,
- un dispositif de traitement automatisé des données, tel qu'un micro-ordinateur, muni de cartes électroniques permettant de commander le flux de données au sein du bus de données et d'une carte électronique ou d'un logiciel capable de traiter mathématiquement les données collectées.

Le ou les boîtier(s) de mesure comprennent avantageusement chacun une ou plusieurs cartes de relais, destinées à activer des électrovannes, et une ou plusieurs cartes de mesure. Ces dernières comportent:

- un système de mesure relié à l'émetteur et au récepteur, et
- un convertisseur digital/analogique permettant de traiter les données venant du bus et ainsi de fixer une tension adéquate à appliquer à l'émetteur afin de maintenir la tension de référence du récepteur.

Avantageusement, un boîtier de mesure comprend, par exemple, dix cartes de mesure et cinq cartes de relais chacune des cartes de mesure étant reliée à un microfermenteur et les cartes de relais étant reliées aux électrovannes.

**Revendications**

1.  Appareil de mesure d'au moins une propriété optique (T, A, F) d'un milieu (1), comprenant:

    - une source de lumière (3) destinée à émettre un faisceau lumineux (11) d'intensité variable vers et dans le milieu (1),
    - des moyens de détection (4) destinée à mesurer l'intensité du faisceau renvoyé (12) par le milieu (1),
    - des moyens de commande (6) de l'intensité du faisceau émis (11) par la source (3),
    - des moyens de stockage destinés à stocker une valeur représentative (V2) d'une intensité nominale du faisceau renvoyé (12),
    - un système de régulation par rétroaction (7) relié aux moyens de commande (6), aux moyens de détection (4) et aux moyens de stockage, agissant sur les moyens de commande (6) de façon à ce que l'intensité mesurée du faisceau renvoyé (12) soit égale à l'intensité nominale, et
    - des moyens de lecture de l'intensité du faisceau émis (11) pour ladite intensité nominale du faisceau renvoyé (12), lesdites intensités du faisceau émis (11) et nominale du faisceau renvoyé (12) étant représentatives de ladite au moins une propriété optique (T, A, F) du milieu (1),

    **caractérisé en ce que** ledit appareil comprend des moyens d'ajustement de l'intensité nominale, reliés auxdits moyens de lecture et de stockage, destinés à ajuster l'intensité nominale de façon que l'intensité stabilisée du faisceau émis (11) soit incluse dans une plage prédéterminée.

2.  Appareil de mesure selon la revendication 1, **caractérisé en ce que** les moyens de stockage sont destinés à stocker une intensité de saturation et **en ce que** les moyens d'ajustement régulent l'intensité nominale à une valeur moindre lorsque l'intensité stabilisée du faisceau émis (11) est supérieure à l'intensité de saturation.

3.  Appareil de mesure selon l'une des revendications 1 ou 2, **caractérisé en ce que** la source de lumière (3) et/ou les moyens de détection (4) sont destinés à mesurer l'intensité du faisceau renvoyé (12) autour d'au moins une longueur d'onde, chacune desdites au moins une propriété optique (T, A, F) du milieu (1) correspondant à l'une desdites au moins une longueur d'onde.

4.  Appareil de mesure selon l'une quelconque des revendications 1 à 3, **caractérisé en ce que** lesdites au moins une propriété optique du milieu (1) comprennent la turbidité (T) du milieu (1).

5.  Appareil de mesure selon la revendication 4, **caractérisé en ce que** la source de lumière (3) et/ou les moyens de détection (4) sont destinés à des mesures en infrarouge.

6.  Appareil de mesure selon l'une quelconque des revendications 1 à 3, **caractérisé en ce que** ladite au moins une propriété optique du milieu (1) comprend l'absorbante (A) du milieu (1).

7.  Appareil de mesure selon l'une quelconque des revendications 1 à 3, **caractérisé en ce que** ladite au moins une propriété optique du milieu (1) comprend la fluorescence (F) du milieu (1).

8.  Appareil de mesure selon l'une quelconque des revendications 1 à 7, **caractérisé en ce qu'**il comprend une unité de traitement (5) reliée aux moyens de commande (6) et de régulation par rétroaction (7) et comportant les moyens de stockage, les moyens de stockage étant destinés à stocker au moins une équation déterminée par une courbe d'étalonnage (101-107) donnant ladite au moins une propriété optique (T, A, F) du milieu ( 1) en fonction de l'intensité du faisceau émis (11) pour respectivement au moins une valeur de l'intensité du faisceau renvoyé (12), et **en ce que** l'unité de traitement extrait ladite au moins une propriété optique (T, A, F) du milieu (1) à partir de l'intensité stabilisée du faisceau émis (11) pour une desdites valeurs de l'intensité nominale, d'une part, et de l'équation correspondant à ladite valeur de l'intensité nominale, d'autre part.

9.  Appareil de mesure selon la revendication 8, **caractérisé en ce que** l'unité de traitement (5) comprend une unité centrale (8) et au moins une carte de conversion analogique/numérique et numérique/analogique, couplant l'unité centrale (8) au moyen de commande (6) et aux moyens de régulation (7).

10. Appareil de mesure selon l'une quelconque des revendications précédentes, **caractérisé en ce qu'**il comporte une horloge, des moyens d'enregistrement dans les moyens de stockage de valeurs représentatives (V1, T, A, F) de ladite au moins une propriété optique du milieu (1), ainsi que des moyens d'activation répétée du système de régulation par rétroaction (7) et des moyens d'enregistrement.

**11.** Appareil de mesure selon l'une quelconque des revendications précédentes, **caractérisé en ce que** le système de régulation par rétroaction (7) et les moyens de commande (6) sont tels que l'égalisation de l'intensité mesurée et de l'intensité nominale du faisceau renvoyé (12) est obtenue de manière continue.

**12.** Microfermenteur (20, 41-48) **caractérisé en ce qu'**il est muni d'un appareil de mesure selon l'une quelconque des revendications 1 à 11.

**13.** Microfermenteur selon la revendication 12, **caractérisé en ce qu'**il comprend:

- un tube (2) fermé par un bouchon (22) destiné à contenir une culture (1),
- une première canalisation (23, 24, 25, 33, 54, 55) destinée à injecter de l'air ou du gaz stériles (14) et du milieu de culture (15) dans la culture (1), et
- une seconde canalisation (27, 37, 57), destinée à récupérer des effluents (17),
- le tube (2) et toutes les canalisations (23-27, 33? 36, 37, 54, 55, 57) étant sous pression.

**14.** Microfermenteur selon l'une des revendications 12 ou 13, **caractérisé en ce qu'**il comprend un système de dilution destiné à diluer une culture et **en ce que** l'appareil de mesure comprend une horloge et une unité de traitement informatique (5) reliée au système de dilution, l'unité de traitement déclenchant cycliquement le système de dilution.

**15.** Microfermenteur selon la revendication 14, **caractérisé en ce que** l'unité de traitement informatique (5) active de manière répétée le système de régulation par rétroaction (7), enregistre dans les moyens de stockage des valeurs représentatives (V1, T, A, F) de ladite au moins une propriété optique, et déclenche le système de dilution lorsqu'elle constate sur une pluralité des dernières valeurs enregistrées une stagnation de ladite au moins une propriété optique (T, A, F) du milieu (1).

**16.** Microfermenteur selon l'une quelconque des revendications 12 à 15, **caractérisé en ce que** les canalisations (23-27, 33, 36, 37, 54, 55, 57) sont raccordées au moyen de connecteurs (200) comportant chacun une partie mâle (201) reliée à une première (211) des canalisations, une partie femelle (202) reliée à une deuxième (214) des canalisations et des moyens de connexion (221, 237) de la partie mâle (201) à la partie femelle (202), chacune desdites parties mâle (201) et la partie femelle( 202) comprenant:

- une extrémité (219, 233) allongée et creuse reliée à ladite canalisation (211, 214) correspondant à ladite partie (201, 202) et communiquant avec ladite canalisation, et
- un fourreau (220, 234) allongé, entourant ladite extrémité (219, 233) et relié à ladite canalisation (211, 214) l'extrémité (219) de la partie mâle (201) étant introduite dans l'extrémité (233) de la partie femelle (202), le fourreau (220) de l'une des parties (201) étant introduit dans le fourreau (234) de l'autre partie (202) et lesdits fourreaux (220, 234) étant fixés l'un à l'autre par les moyens de connexions (221, 237).

**17.** Microfermenteur selon la revendication 16, **caractérisé en ce que** le fourreau (220) de la partie mâle (201) est introduit dans le fourreau (234) de la partie femelle (202).

**18.** Microfermenteur selon l'une des revendications 16 ou 17, **caractérisé en ce que** les moyens de connexion (221, 237) comprennent une bague de serrage (221) reliée à la canalisation (211) correspondant au fourreau (220) récepteur et un système de fixation (237) prévu sur le fourreau (234) introduit dans le fourreau (220) récepteur et coopérant avec ladite bague (221).

**19.** Procédé de mesure d'au moins une propriété optique (T, A, F) d'un milieu (1), dans lequel:

- on émet un faisceau lumineux (11) d'intensité variable vers le milieu (1), et
- on mesure l'intensité du faisceau renvoyé (12) par le milieu (1),
- on ajuste l'intensité du faisceau émis (11) de façon à ce que l'intensité du faisceau renvoyé (12) soit égale à une intensité nominale fixée,
- on lit l'intensité stabilisée du faisceau émis (11) ainsi obtenue, et
- on rapporte une valeur représentative de l'intensité stabilisée à une équation déterminée par une courbe (101-117) donnant ladite au moins une propriété optique (T, A, F) en fonction de la valeur représentative de l'intensité stabilisée du faisceau émis (11) pour ladite intensité nominale du faisceau renvoyé (12), de façon

## EP 1 034 422 B1

à extraire ladite au moins une propriété optique (T, A, F) du milieu (1),

**caractérisé en ce qu'**on ajuste l'intensité nominale de façon que l'intensité stabilisée du faisceau émis (11) soit incluse dans une plage déterminée.

### Claims

1. Apparatus for measuring at least one optical property (T, A, F) of a medium (1), comprising:

   - a light source (3) which transmits a light beam (11) of variable intensity towards and into the medium (1),
   - detection means (4) to measure the intensity of the beam (12) reflected by the medium (1),
   - means of controlling (6) the intensity of the beam (11) transmitted by the source (3),
   - storage means which store a representative value (V2) of a nominal intensity of the reflected beam (12),
   - a feedback regulating system (7) linked to the control means (6), the detection means(4) and the storage means, operating on the control means (6) so that the measured intensity of the reflected beam (12) is equal to the nominal intensity, and
   - means of reading the intensity of the transmitted beam (11), for said nominal intensity of the reflected beam (12), said intensity of the transmitted beam (11) and said nominal intensity of the reflected beam (12) being representative of at least one of said optical properties (T, A, F) of the medium (1), **characterised in that** said apparatus comprises means for adjusting the nominal intensity, linked to said reading means and storage means, designed to adjust the nominal intensity so that the stabilised intensity of the transmitted beam (11) falls within a predetermined range.

2. Measurement apparatus according to claim 1, **characterised in that** the storage means are designed to store a saturation intensity and **in that** the adjustment means regulate the nominal intensity to a lower value when the stabilised intensity of the transmitted beam (11) is higher than the saturation intensity.

3. Measurement apparatus according to one of claims 1 or 2, **characterised in that** the light source (3) and/or the detection means (4) are designed to measure the intensity of the reflected beam (12) around at least one wavelength, each of said optical property or properties (T, A, F) of the medium (1) corresponding to at least one of said wavelengths.

4. Measurement apparatus according to any one of claims 1 to 3, **characterised in that** one of said optical properties of the medium (1) comprises the turbidity (T) of the medium (1).

5. Measurement apparatus according to claim 4, **characterised in that** the light source (3) and/or the detection means (4) are intended for measurements in the infrared.

6. Measurement apparatus according to any one of claims 1 to 3, **characterised in that** one of said optical properties of the medium (1) comprises the absorbance (A) of the medium (1).

7. Measurement apparatus according to any one of claims 1 to 3, **characterised in that** one of said optical properties of the medium (1) comprises the fluorescence (F) of the medium (1).

8. Measurement apparatus according to any one of claims 1 to 7, **characterised in that** it comprises a processing unit (5) linked to the control means (6) and the feedback regulating system (7) and comprising storage means, the storage means being designed to store at least one equation determined by a calibration curve (101-117) giving said optical property or properties (T, A, F) of the medium (1) as a function of the intensity of the transmitted beam (11) for respectively at least one value of the intensity of the reflected beam (12), and **in that** the processing unit calculates said optical property or properties (T, A, F) of the medium (1) from the stabilised intensity of the transmitted beam (11) for one of said values of the nominal intensity, on the one hand, and the equation corresponding to said value of the nominal intensity, on the other.

9. Measurement apparatus according to claim 8, **characterised in that** the processing unit (5) comprises a central processing unit (8) and at least one card for analog/digital and digital/analog conversion, coupling the central processing unit (8) to the control means (6) and the regulation means (7).

17

10. Measurement apparatus according to any one of the preceding claims, **characterised in that** it comprises a clock, recording means in the means of storage of representative values (V1, T, A, F) of said optical property or properties of the medium (1), and means of repeated activation of the feedback regulating system (7) and the recording means.

11. Measurement apparatus according to any one of the preceding claims, **characterised in that** the feedback regulating system (7) and the control means (6) are such that the equalisation of the measured intensity and the nominal intensity of the reflected beam (12) is obtained continuously.

12. Micro-bioreactor (20, 41-48) **characterised in that** it is provided with a measurement apparatus according to any one of claims 1 to 11.

13. Micro-bioreactor according to claim 12, **characterised in that** it comprises:

- a tube (2) closed by a stopper (22), intended to contain a culture (1),
- a first pipe (23, 24, 25, 33, 54, 55), to inject sterile air or gas (14) and the culture medium (15) into the culture (1), and
- a second pipe (27, 37, 57), to remove the effluents (17),

the tube (2) and all the pipes (23-27, 33, 36, 37, 54, 55, 57) being under pressure.

14. Micro-bioreactor according to one of claims 12 or 13, **characterised in that** it comprises a dilution system designed to dilute a culture and **in that** the measurement apparatus comprises a clock and a computer processing unit (5) linked to the dilution system, the processing unit cyclically triggering the dilution system.

15. Micro-bioreactor according to claim 14, **characterised in that** the computer processing unit (5) repeatedly activates the feedback regulating system (7), records the representative values (V1, T, A, F) of said optical property or properties in the storage means, and triggers the dilution system when it observes a stagnation of said optical property or properties (T, A, F) of the medium (1) over a plurality of the latest recorded values.

16. Micro-bioreactor according to any one of claims 12 to 15, **characterised in that** the pipes (23-27, 33, 36, 37, 54, 55, 57) are connected by means of connectors (200) each comprising a male part (201) connected to a first pipe (211), a female part (202) connected to a second pipe (214), and means of connection (221, 237) of said male part (201) and said female part (202), each of said male (201) and female (202) parts comprising:

- a tapered and hollow end (219, 233) connected to said pipe (211, 214) corresponding to said part (201, 202) and communicating with said pipe, and
- a tapered sleeve (220, 234), surrounding said end (219, 233) and connected to said pipe (211, 214), the end (219) of the male part (201) being introduced into the end (233) of the female part (202), the sleeve (220) of one of the parts (201) being introduced into the sleeve (234) of the other part (202) and said sleeves (220, 234) being fixed to each other by the connection means (221, 237).

17. Micro-bioreactor according to claim 16, **characterised in that** the sleeve (220) of the male part (201) is introduced into the sleeve (234) of the female part (202).

18. Micro-bioreactor according to one of claims 16 or 17, **characterised in that** the connection means (221, 237) comprise a locking ring (221) linked to the pipe (211) corresponding to the receptor sleeve (220) and a fixing system (237) provided on the sleeve (234) introduced into the receptor sleeve (220) and engaging said ring (221).

19. Method for measuring at least one optical property (T, A, F) of a medium (1), wherein :

- a light beam (11) of variable intensity is transmitted towards the medium (1),
- the intensity of the beam (12) reflected by the medium (1) is measured,
- the intensity of the transmitted beam (11) is adjusted so that the intensity of the reflected beam (12) is equal to a fixed nominal value,
- the stabilised intensity of the transmitted beam (11) thus obtained is read, and
- a value representative of the stabilised intensity is related to an equation determined by a curve (101-117) giving said optical property or properties (T, A, F) as a function of the representative value of the stabilised intensity of the transmitted beam (11) for said nominal intensity of the reflected beam (12), so as to calculate said optical property or properties (T, A, F) of the medium (1), **characterised in that** the nominal intensity is adjusted so that the stabilised intensity of the transmitted beam (11) is within a predetermined range.

**Patentansprüche**

1. Vorrichtung zur Messung wenigstens einer optischen Eigenschaft (T, E, F) eines Mediums (1), umfassend:

   - eine Lichtquelle (3) zur Emission eines Lichtbündels (11) mit variabler Intensität auf und in das Medium (1);

   - Nachweiseinrichtungen (4) zur Messung der Intensität des vom Medium (1) zurückgeworfenen Lichtbündels (12);

   - Steuereinrichtungen (6) für die Intensität des von der Quelle (3) emittierten Lichtbündels (11);

   - Speichereinrichtungen zum Speichern eines Wertes (V2), der eine nominelle Intensität des zurückgeworfenen Lichtbündels (12) darstellt;

   - eine Regeleinrichtung (7), die mit den Steuereinrichtungen (6), den Nachweiseinrichtungen (4) und den Speichereinrichtungen verbunden ist und das so auf die Steuereinrichtungen (6) einwirkt, dass die gemessene Intensität des zurückgeworfenen Lichtbündels (12) gleich der nominellen Intensität ist; und

   - Leseeinrichtungen für die Intensität des emittierten Lichtbündels (11) bei der nominellen Intensität des zurückgeworfenen Lichtbündels (12), wobei die Intensität des emittierten Lichtbündels (11) und die nominelle Intensität des zurückgeworfenen Lichtbündels (12) repräsentativ für die wenigstens eine optische Eigenschaft (T, E, F) des Mediums (1) sind;

   **dadurch gekennzeichnet, dass** die Vorrichtung Einrichtungen zur Einstellung der nominellen Intensität umfasst, die mit den Leseeinrichtungen und Speichereinrichtungen verbunden sind und dazu dienen, die nominelle Intensität so einzustellen, dass die stabilisierte Intensität des emittierten Lichtbündels (11) in einem vorbestimmten Bereich liegt.

2. Messvorrichtung gemäß Anspruch 1, **dadurch gekennzeichnet, dass** die Speichereinrichtungen dazu dienen, eine Sättigungsintensität zu speichern, und dass die Einstellungseinrichtungen die nominelle Intensität auf einen geringeren Wert regeln, während die stabilisierte Intensität des emittierten Lichtbündels (11) oberhalb der Sättigungsintensität liegt.

3. Messvorrichtung gemäß einem der Ansprüche 1 oder 2, **dadurch gekennzeichnet, dass** die Lichtquelle (3) und/oder die Nachweiseinrichtungen (4) zur Messung der Intensität des zurückgeworfenen Lichtbündels (12) in der Nähe wenigstens einer Wellenlänge dienen, wobei die optische Eigenschaft bzw. jede der optischen Eigenschaften (T, E, F) des Mediums (1) jeweils einer der Wellenlängen entspricht.

4. Messvorrichtung gemäß einem der Ansprüche 1 bis 3, **dadurch gekennzeichnet, dass** die wenigstens eine optische Eigenschaft des Mediums (1) die Trübung (T) des Mediums (1) umfasst.

5. Messvorrichtung gemäß Anspruch 4, **dadurch gekennzeichnet, dass** die Lichtquelle (3) und/oder die Nachweiseinrichtungen (4) zur Messung im Infrarot dienen.

6. Messvorrichtung gemäß einem der Ansprüche 1 bis 3, **dadurch gekennzeichnet, dass** die wenigstens eine optische Eigenschaft des Mediums (1) die Extinktion (E) des Mediums (1) umfasst.

7. Messvorrichtung gemäß einem der Ansprüche 1 bis 3, **dadurch gekennzeichnet, dass** die wenigstens eine optische Eigenschaft des Mediums (1) die Fluoreszenz (F) des Mediums (1) umfasst.

8. Messvorrichtung gemäß einem der Ansprüche 1 bis 7, **dadurch gekennzeichnet, dass** er eine Verarbeitungseinheit (5) umfasst, die mit den Steuereinrichtungen (6) und der Regeleinrichtung (7) verbunden ist und Speichereinrichtungen umfasst, wobei die Speichereinrichtungen dazu dienen, wenigstens eine Gleichung zu speichern, die durch eine Eichkurve (101-107) bestimmt ist, welche die wenigstens eine optische Eigenschaft (T, E, F) des Mediums (1) als Funktion der Intensität des emittierten Lichtbündels (11) für jeweils wenigstens einen Wert der Intensität des zurückgeworfenen Lichtbündels (12) wiedergibt, und dass die Verarbeitungseinheit die wenigstens eine optische Eigenschaft (T, E, F) des Mediums (1) einerseits aus der stabilisierten Intensität des emittierten Lichtbündels (11) für einen der Werte der nominellen Intensität und andererseits aus der Gleichung, die dem Wert der nominellen Intensität entspricht, entnimmt.

9. Messvorrichtung gemäß Anspruch 8, **dadurch gekennzeichnet, dass** die Verarbeitungseinheit (5) eine Zentraleinheit (8) und wenigstens eine Analog/Digital- und Digital/Analog-Wandlerkarte, die die Zentraleinheit (8) mit der Steuereinrichtung (6) und mit den Regeleinrichtungen (7) koppelt, umfasst.

10. Messvorrichtung gemäß einem der vorstehenden Ansprüche, **dadurch gekennzeichnet, dass** sie einen Taktgeber, Aufzeichnungseinrichtungen in den Speichereinrichtungen für die repräsentativen Werte (V1, T, E, F) der wenigstens einen optischen Eigenschaft des Mediums (1) sowie Einrichtungen zur wiederholten Aktivierung der Regeleinrichtung (7) und der Aufzeichnungseinrichtungen umfasst.

11. Messvorrichtung gemäß einem der vorstehenden Ansprüche, **dadurch gekennzeichnet, dass** die Regeleinrichtung (7) und die Steuereinrichtungen (6) so gestaltet sind, dass der Abgleich der gemessenen Intensität und der nominellen Intensität des zurückgeworfenen Lichtbündels (12) kontinuierlich erreicht wird.

12. Mikrofermenter (20, 41-48), **dadurch gekennzeichnet, dass** er mit einer Messvorrichtung gemäß einem der Ansprüche 1 bis 11 ausgestattet ist.

13. Mikrofermenter gemäß Anspruch 12, **dadurch gekennzeichnet, dass** er folgendes umfasst:

    - ein Rohr (2), das mit einem Stopfen (22) verschlossen ist und dazu dient, eine Kultur (1) zu enthalten;

    - ein erstes Leitungssystem (23, 24, 25, 33, 54, 55) zum Einleiten von Luft oder sterilem Gas (14) und Kulturmedium (15) in die Kultur (1); und

    - ein zweites Leitungssystem (27, 37, 57) zur Rückführung der Ausflüsse (17);

    - wobei das Rohr (2) und alle Leitungssysteme (23-27, 33, 36, 37, 54, 55, 57) unter Druck stehen.

14. Mikrofermenter gemäß einem der Ansprüche 12 oder 13, **dadurch gekennzeichnet, dass** er ein Verdünnungssystem zum Verdünnen einer Kultur umfasst und dass die Messvorrichtung einen Taktgeber und eine mit dem Verdünnungssystem verbundene Datenverarbeitungseinheit (5) umfasst, wobei die Verarbeitungseinheit das Verdünnungssystem cyclisch auslöst.

15. Mikrofermenter gemäß Anspruch 14, **dadurch gekennzeichnet, dass** die Datenverarbeitungseinheit (5) das Regelsystem (7) wiederholt aktiviert, in den Speichereinrichtungen repräsentative Werte (V1, T, E, F) der wenigstens einen optischen Eigenschaft aufzeichnet und das Verdünnungssystem auslöst, wenn sie in einer Menge der letzten aufgezeichneten Werte eine Stagnation der wenigstens einen optischen Eigenschaft (T, E, F) des Mediums (1) feststellt.

16. Mikrofermenter gemäß einem der Ansprüche 12 bis 15, **dadurch gekennzeichnet, dass** die Leitungssysteme (23-27, 33, 36, 37, 54, 55, 57) mittels Verbindungsteilen (200) miteinander verbunden sind, die jeweils einen männlichen Teil (201), der mit einem ersten (211) der Leitungssysteme verbunden ist, einen weiblichen Teil (202), der mit einem zweiten (214) der Leitungssysteme verbunden ist, und Verbindungseinrichtungen (221, 237) des männlichen Teils (201) mit dem weiblichen Teil (202) umfassen, wobei jeder der männlichen Teile (201) und der weibliche Teil (202) folgendes umfassen:

    • ein längliches und hohles Ende (219, 233), das mit dem Leitungssystem (211, 214) verbunden ist und das dem Teil (201, 202) entspricht und mit dem Leitungssystem in Verbindung steht; und

    • eine längliche Hülse (220, 234), die das Ende (219, 233) umgibt und mit dem Leitungssystem (211, 214) verbunden ist, wobei das Ende (219) des männlichen Teils (201) in das Ende (233) des weiblichen Teils (202) eingeführt wird, die Hülse (220) eines der Teile (201) in die Hülse (234) des anderen Teils (202) eingeführt wird und die Hülsen (220, 234) durch die Verbindungseinrichtungen (221, 237) aneinander befestigt sind.

**17.** Mikrofermenter gemäß Anspruch 16, **dadurch gekennzeichnet, dass** die Hülse (220) des männlichen Teils (201) in die Hülse (234) des weiblichen Teils (202) eingeführt wird.

**18.** Mikrofermenter gemäß einem der Ansprüche 16 oder 17, **dadurch gekennzeichnet, dass** die Verbindungseinrichtungen (221, 237) einen Klemmring (221), der mit dem Leitungssystem (211) verbunden ist, das der aufnehmenden Hülse (220) entspricht, und ein Befestigungssystem (237), das auf der Hülse (234), die in die aufnehmende Hülse (220) eingeführt wird, vorgesehen ist und mit dem Klemmring (221) zusammenarbeitet, umfassen.

**19.** Verfahren zur Messung wenigstens einer optischen Eigenschaft (T, E, F) eines Mediums (1), wobei:

ein Lichtbündel (11) mit variabler Intensität auf das Medium (1) emittiert wird und

die Intensität des vom Medium (1) zurückgeworfenen Lichtbündels (12) gemessen wird;

die Intensität des emittierten Lichtbündels (11) so eingestellt wird, dass die Intensität des zurückgeworfenen Lichtbündels (12) gleich einer festgelegten nominellen Intensität ist;

die so erhaltene stabilisierte Intensität des emittierten Lichtbündels (11) gelesen wird; und

ein Wert, der der stabilisierten Intensität entspricht, mit einer Gleichung in Bezug gesetzt wird, die durch eine Kurve (101-117) bestimmt ist, welche die wenigstens eine optische Eigenschaft (T, E, F) als Funktion des Werts, der der stabilisierten Intensität des emittierten Lichtbündels (11) entspricht, für die nominelle Intensität des zurückgeworfenen Lichtbündels (12) wiedergibt, so dass man daraus die wenigstens eine optische Eigenschaft (T, E, F) des Mediums (1) erhält;

**dadurch gekennzeichnet, dass** die nominelle Intensität so eingestellt wird, dass die stabilisierte Intensität des emittierten Lichtbündels (11) in einem vorbestimmten Bereich liegt.

FIG.1

FIG.2

FIG.3

FIG.13

FIG.4

FIG.5

FIG.6

FIG.7

FIG.8

FIG.9

FIG.10

FIG.11

EP 1 034 422 B1

FIG.12

FIG.14

FIG.15